# EUROPÄISCHE PATENTSCHRIFT

(11) **EP 4 214 679 B1**
(45) Veröffentlichungstag und Bekanntmachung des Hinweises auf die Patenterteilung: **02.09.2026**
(21) Anmeldenummer: 21778406.5
(22) Anmeldetag: 17.09.2021
(51) Int. Cl.: G06T 7/50, G06T 7/30, G01B 9/027, G01B 11/24, G01B 11/25, G02B 23/24, H04N 13/221

(54) **OPTISCHE VORRICHTUNG UND VERFAHREN ZUR UNTERSUCHUNG EINES GEGENSTANDES**
OPTICAL DEVICE AND METHOD FOR EXAMINING AN OBJECT
DISPOSITIF OPTIQUE ET PROCÉDÉ POUR INSPECTER UN OBJET

(30) Priorität: 21.09.2020 DE 102020124521
(43) Veröffentlichungstag der Anmeldung: 26.07.2023
(73) Patentinhaber: AKMIRA OPTRONICS GMBH, 14480 Potsdam (DE)
(72) Erfinder: KNÜTTEL, Alexander, 14480 Potsdam (DE)
(74) Vertreter: Schulz Junghans Patentanwälte PartGmbB
(86) Internationale Anmeldenummer: PCT/EP2021/075619
(87) Internationale Veröffentlichungsnummer: WO 2022/058498

(56) Entgegenhaltungen:
- US-A1- 2018 211 373
- US-A1- 2019 231 220
- US-A1- 2019 281 272

## Beschreibung

Die vorliegende Erfindung betrifft eine optische Vorrichtung und ein Verfahren zur Untersuchung eines Gegenstandes mit insbesondere sichtbarem Objektlicht zur Erstellung eines 3D-Oberflächendatensatzes des Gegenstandes, wobei der Datensatz eine laterale Information und eine Tiefeninformation umfasst.

Die Vorrichtung ist vorzugsweise handhaltbar und/oder handgeführt, wobei sie baulich kompakt ist. Die Vorrichtung ist oder bildet beispielsweise eine endoskopische Vorrichtung, ein tragbares Kommunikationsgerät oder ein Head-mounted-Gerät und/oder ist von einer solchen bzw. einem solchen umfasst.

Die Erfindung bezieht sich auf das Gebiet der digitalen Bilderzeugung und -verarbeitung, bei dem Untersuchungslicht auf einen einzelne Pixel aufweisenden Bildsensor einer Sensoreinheit fällt. Bildsignale des Bildsensors werden einer Datenverarbeitungseinheit der Vorrichtung zugeführt und von dieser zur Erstellung von Datensätzen verarbeitet. Eine Anzeigeeinheit zur Darstellung einer Bildinformation der Datensätze und/oder eine Speichereinheit zur Speicherung der Datensätze kann vorgesehen sein.

Bekannt ist es, zweidimensionale Bilder mittels herkömmlicher (digitaler) Fotografie zu erstellen. Vergrößerungen können unter Einsatz refraktiver oder reflektiver Optik erzielt werden. Nachteilig ist hierbei der erforderliche Platzbedarf, verbunden mit Vergrößerung der Abmessungen der Vorrichtung. Die Auflösung wird durch die numerische Apertur bestimmt.

Um einen 3D-Oberflächendatensatz mit einer Tiefeninformation bereitzustellen, kann beispielsweise Stereoskopie eingesetzt werden. Hierbei werden zwei Aufnahmen der Szene unter unterschiedlichem Winkel erstellt. Nachteilig ist hierbei, dass zum Erzielen einer hohen Tiefenauflösung ein großer Abstand der Bildsensoren (Baseline) erforderlich ist. Unabhängig hiervon können Artefakte auftreten, beispielsweise bei Kantenverläufen.

Eine Tiefeninformation kann zum Beispiel auch unter Einsatz von plenoptischen Kamerasystemen bereitgestellt werden. Über Multilinsenoptiken werden Wellenfronten mit unterschiedlichen lokalen Phasen erzeugt, was jedoch einen erheblichen rechnerischen Aufwand zur Rekonstruktion der Szene erfordert. Fehler können insbesondere bei schwachem Lichteinfall auftreten. Vorrichtungen und Verfahren zur Erzeugung digitaler Bilddatensätze sind beispielsweise in der US 10,536,684 B2, der US 8,456,517 B2, der US 6,664,529 B2 und der US 7,295,324 B2 beschrieben.

US-Patent 2019/281272 A1 (BABAYOFF NOAM [IL]) 12. September 2019, offenbart eine Vorrichtung zur Bestimmung der Oberflächentopologie und der zugehörigen Farbe einer Struktur, die einen Scanner zur Erfassung von Tiefendaten für Punkte entlang einer zweidimensionalen Anordnung, die im Wesentlichen orthogonal zur Tiefenrichtung verläuft, sowie eine Bildaufnahmeeinrichtung zur Erfassung von Farbdaten für jeden der Punkte der Anordnung umfasst, wobei die räumliche Anordnung der Vorrichtung in Bezug auf die Struktur im Wesentlichen unverändert bleibt. Ein Prozessor kombiniert die Farbdaten und Tiefendaten für jeden Punkt in der Anordnung und liefert dadurch ein dreidimensionales virtuelles Farbmodell der Oberfläche der Struktur.

US-Patent 2019/231220 A1 (REFAI HAKKI [US] ET AL) 1. August 2019, offenbart ein System für dreidimensionale Bildgebung, Modellierung, Kartierung und/oder Steuerungsfunktionen in kompakter Bauweise, das sich für die Integration in und/ oder die Erweiterung von robotergestützten, laparoskopischen und endoskopischen Operationssystemen eignet. Die Systeme umfassen eine optische Quelle, die so konfiguriert ist, dass sie auf eine Operations- oder Endoskopieumgebung projiziert, sowie eine Kamera, die so positioniert ist, dass sie ein Bild der Operations- oder Endoskopieumgebung aufnimmt.

US-Patent US 2018/211373 A1 (STOPPA MICHELE [US] ET AL) 26. Juli 2018, offenbart ein Verfahren zur Fehlererkennung auf der Oberfläche eines Objekts. Das Verfahren umfasst die Erfassung von Teilpunktwolken eines Objekts aus verschiedenen Positionen relativ zum Objekt mittels Tiefenkameras. Die Teilpunktwolken werden von einem Prozessor zusammengeführt, um eine zusammengeführte Punktwolke zu erzeugen. Ein dreidimensionales (3D) Multi-View-Modell des Objekts wird vom Prozessor berechnet, an einem Referenzmodell ausgerichtet und schließlich mit dem Referenzmodell verglichen, um Unterschiede zwischen entsprechenden Bereichen des 3D-Multi-View-Modells und des Referenzmodells zu berechnen.

Aufgabe der vorliegenden Erfindung ist es, eine optische Vorrichtung und ein Verfahren zur Untersuchung eines Gegenstandes mit verbesserten Abbildungseigenschaften bereitzustellen.

Diese Aufgabe wird durch eine erfindungsgemäße optische Vorrichtung zur Untersuchung eines Gegenstandes gelöst, umfassend ein Gehäuse, eine im Gehäuse angeordnete optische Einheit für einfallendes Licht, eine Sensoreinheit mit mindestens einem im Gehäuse angeordneten Bildsensor, eine Datenverarbeitungseinheit, die mit der Sensoreinheit gekoppelt ist und Bildsignale des mindestens einen Bildsensors auswertet, eine zumindest teilweise am oder im Gehäuse angeordnete Beleuchtungseinheit zur Emission von Rasterlicht in Richtung des Gegenstandes, wobei mittels vom Gegenstand reflektierten Rasterlicht und gehäuseinternem Referenzlicht durch digitale optische Holografie ein dreidimensionales Punktraster und diesbezügliche Referenzdatensätze indikativ für eine Lateralinformation und eine Tiefeninformation bereitgestellt werden, wobei über die Vorrichtung eine Relativbewegung der Vorrichtung und des Gegenstandes erfasst und eine diesbezügliche Bewegungsinformation in Lateral- und/oder Tiefenrichtung erstellt wird, wobei vom Gegenstand ausgehendes Objektlicht erfasst und zeitlich aufeinanderfolgend ein jeweiliger Bilddatensatz erstellt wird, der relativ zum Referenzdatensatz zur Erstellung eines 3D-Oberflächendatensatzes registriert wird, wobei einander überlappende Bereiche von zwei oder mehr aufeinanderfolgenden Bilddatensätzen anhand der Bewegungsinformation identifiziert werden und die überlappenden Bereiche durch Integration der diesbezüglichen Bildsignale geglättet werden, insbesondere ohne Verlust an Detailtreue.

Bei der erfindungsgemäßen Vorrichtung wird zur Erstellung einer Lateral- und/oder Tiefeninformation, die insbesondere die Oberfläche des Gegenstandes beschreibt, digitale optische Holografie eingesetzt. Zu diesem Zweck kann ein Punktraster über Rasterlicht auf den Gegenstand emittiert und dem mindestens einen Bildsensor über die optische Einheit zugeführt werden. Unter Nutzung von Referenzlicht, das auf dem Bildsensor mit dem Rasterlicht inteferiert, kann auf diese Weise ein optisches Gitter am Gegenstand gewissermaßen "angeheftet" werden, welches Stützstellen für die Registrierung der Bilddatensätze bildet. Der Bilddatensatz kann mittels Untersuchungslicht, das über die optische Einheit auf den mindestens einen Bildsensor abgebildet wird, erstellt werden. Insbesondere ist der Bilddatensatz vorzugsweise höher aufgelöst als der Referenzdatensatz und lediglich durch die optische Auflösung des Systems begrenzt. Der Bilddatensatz kann rechnerisch über das optische Gitter des Referenzdatensatzes gezogen und dadurch ein 3D-Oberflächendatensatz erstellt werden. Die Vorrichtung ist insbesondere ein optischer 3D-Scanner oder bildet einen solchen aus.

Bei der erfindungsgemäßen Vorrichtung besteht insbesondere der Vorteil, dass eine Relativbewegung der Vorrichtung und des Gegenstandes mittels der Vorrichtung selbst erfasst werden kann. Eine diesbezügliche Bewegungsinformation in Lateral- und/oder Tiefenrichtung kann bereitgestellt werden. Basierend auf der Bewegungsinformation können aufeinanderfolgende Bilddatensätze räumlich in Übereinstimmung gebracht werden. Hierbei fließt die Überlegung ein, dass die von der Vorrichtung beobachtbare Szene bei einer Relativbewegung mitbewegt ist. Es besteht daher die Möglichkeit, überlappende Bereiche von zwei oder mehr Bilddatensätzen zu identifizieren. Zwangsläufig in den Bilddatensätzen enthaltenes Rauschen kann durch Integration der Bilddatensätze reduziert werden und dadurch die überlappenden Bereiche der Bilddatensätze geglättet werden. Dies ist vorzugsweise möglich, ohne dass Objektauflösung und damit Detailtreue verloren geht. Infolgedessen wird das Signal-zu-Rausch-Verhältnis verbessert (S/N-Verhältnis). Auf diese Weise kann rechnerisch mit verhältnismäßig geringem Aufwand die Abbildungsqualität der Vorrichtung ohne Verlust an Objektauflösung und damit räumlicher Information vorzugsweise bis an die Auflösungsgrenze verbessert werden und ein hochaufgelöster 3D-Oberflächendatensatz erstellt werden. Beispielsweise kann vorzugsweise eine hohe Kantentreue ohne Verlust an höheren Raumfrequenzen erzielt werden.

Günstigerweise kann der 3D-Oberflächendatensatz, wenn rauschfrei oder im Wesentlichen rauschfrei, mittels Entfaltungsalgorithmen zur Erzeugung von "Superresolution"-Bildern weiterverarbeitet werden. Alternativ oder ergänzend sind beispielsweise "Superresolution"-Bilder durch Interpolation innerhalb der 3D-Oberflächendatensätze möglich.

In die Erfindung fließt die Überlegung mit ein, dass bei insbesondere handhaltbaren und/oder handgeführten Vorrichtungen eine Relativbewegung zum Gegenstand häufig bereits aufgrund kaum vermeidbarer Zitterbewegungen des Benutzers auftritt. Es besteht daher insbesondere die Möglichkeit, die Zitterbewegung selbst als Grundlage für die Auswertung der Bewegungsinformation heranzuziehen. Vorteilhaft ist in diesem Zusammenhang insbesondere auch, dass die Bewegungsinformation mittels der Vorrichtung selbst gewonnen werden kann, deren Komponenten zum Teil in einem gemeinsamen Gehäuse angeordnet sind. Dies wiederum begünstigt eine kompakte Bauform der Vorrichtung mit dem Ziel der Handhaltung und/oder Handführung.

Bei der Relativbewegung kann vorgesehen sein, dass diese auf eine rein laterale Bewegung oder auf eine Bewegung allein in Tiefenrichtung (axial) beschränkt ist. Die Erfindung ist jedoch hierauf nicht limitiert. Es kann insbesondere eine 3D-Relativbewegung erkannt und angewendet werden.

"Glätten" kann im vorliegenden Fall derart aufgefasst werden, dass Signalbeiträge aus den einander überlappenden Bereichen der zwei oder mehr aufeinanderfolgenden Bilddatensätze zeitlich integriert werden. Eine Mittelung kann vorgesehen sein, wobei keine "Weichzeichnung" mit Verlust von Kantentreue durchgeführt wird, um die Objektauflösung verlustfrei zu erhalten.

Es versteht sich, dass die Verarbeitung von Bildsignalen des mindestens einen Bildsensors, die Erstellung und/oder Verrechnung von Bilddatensätzen sowie die Berechnung von Bewegungsinformationen von der Datenverarbeitungseinheit durchführbar ist. Dies ist nachfolgend zur Erleichterung des Leseflusses nicht im Einzelnen erwähnt. Es wird jedoch hervorgehoben, dass die Datenverarbeitungseinheit so ausgebildet und programmiert ist, die diesbezüglichen Operationen vornehmen zu können.

Vorgesehen sein kann insbesondere, dass überlappende Bereiche der zwei oder mehr Bilddatensätze durch inverse Bewegung rechnerisch übereinandergelegt werden. Ist die Relativbewegung bekannt, können nachfolgende Bilddatensätze um einen inversen Betrag rechnerisch zurückgeschoben und dadurch mit einem vorangegangenen Bilddatensatz überlagert werden. Der Überlapp zwischen zwei Bereichen ist umso größer, je geringer die Relativbewegung ist.

Eine Einzelbelichtungszeit für einen jeweiligen Bilddatensatz ist vorzugsweise kleiner als ein Untersuchungszeitraum, über den die Bilddatensätze integriert werden.

In der Praxis erweist sich beispielsweise eine Framerate von ungefähr 250 Hz oder mehr als vorteilhaft, vorzugsweise bis ungefähr 1 kHz und darüber.

Günstig ist es, wenn eine Mehrzahl von 3D-Oberflächendatensätzen zu einer Gesamtszene des Gegenstandes zusammengesetzt wird, wobei Grenzen zwischen 3D-Oberflächendatensätzen anhand der Bewegungsinformation ermittelt werden. Über einen längeren Zeitraum können dadurch insbesondere unterschiedliche laterale Szenen aneinandergesetzt werden (Stitching). Im Gegensatz dazu kann eine derartige Gesamtszene bei Einzelaufnahmen insbesondere mit begrenzter Apertur bei herkömmlichen Vorrichtungen nicht erstellt werden. Vorzugsweise weisen die zusammengesetzten Einzelszenen keinen oder nur geringen Versatz zueinander auf.

Zur Ermittlung der Relativbewegung wird mindestens ein vom Rasterlicht auf dem Gegenstand erzeugter Leuchtfleck des Punktrasters mit Specklemuster zeitabhängig untersucht, wobei eine Verschiebung des Specklemusters innerhalb eines Leuchtflecks festgestellt und hieraus eine Bewegungsinformation in Lateralrichtung abgeleitet wird. An der in der Praxis typischerweise nicht perfekt glatten Oberfläche des Gegenstandes kann über multiple Interferenzen ein Specklemuster entstehen. Bewegt sich der Leuchtfleck (Spot) des Rasterlichts relativ zum Gegenstand, kann durch Korrelation aufeinanderfolgender Darstellungen des Leuchtflecks mit Specklemuster (Speckle-Korrelation) die Bewegungsinformation in Lateralrichtung mit hoher Genauigkeit ermittelt werden, weil das Specklemuster ortsfest zum Gegenstand bleibt.

Insbesondere können eine Mehrzahl von Leuchtflecken (Spots) vorzugsweise synchron untersucht werden. Beispielsweise können die unterschiedlichen Spots verschiedenartige laterale Geschwindigkeiten bei bewegten ("atmenden") Gegenständen liefern. Dies macht den Einsatz der Vorrichtung insbesondere im medizintechnischen Umfeld interessant, intrakorporal und/oder extrakorporal.

Günstigerweise kann eine Entfaltungsoperation am 3D-Oberflächendatensatz durchgeführt werden. Hierauf wurde bereits vorstehend eingegangen.

Das Rasterlicht ist oder umfasst beispielsweise Infrarotlicht und/oder Licht des sichtbaren Spektrums. Beispielsweise kommt für Rasterlicht ein Wellenlängenfenster von ungefähr 700 nm bis 1300 nm zum Einsatz. Das Spektrum des Rasterlichts kann sich bis zu Wellenlängen von weniger als 700 nm in das sichtbare Spektrum erstrecken.

Das Rasterlicht und das Referenzlicht sind insbesondere kohärent. Die Beleuchtungseinheit umfasst zu diesem Zweck bevorzugt eine Laserlichtquelle, die vorzugsweise im Gehäuse integriert ist. Ein Strahlteilerelement kann Raster- und Referenzlicht trennen, zur Einkopplung in einen jeweils vorgesehenen optischen Pfad, zum Beispiel über Wellenleiter.

Es versteht sich, dass das Referenzlicht dasselbe Spektrum aufweisen sollte wie das Rasterlicht.

Das Rasterlicht und das Referenzlicht weisen vorzugsweise ein Spektrum einer Mehrzahl diskreter Wellenlängen auf. Es sind hierbei mindestens zwei Wellenlängen vorgesehen, vorzugsweise mehrere Wellenlängen. Beispielsweise werden innerhalb eines Wellenlängenfensters von ungefähr 10 nm mehrere Wellenlängen ungefähr im nm-Abstand eingesetzt.

Eine Bewegungsinformation in Tiefenrichtung und/oder eine absolute Tiefeninformation ist vorzugsweise anhand von Phasendifferenzen der multiplen Wellenlängen ermittelbar ist. Durch die Mehrzahl der Wellenlängen besteht vorzugsweise der Vorteil, dass eine absolute Tiefeninformation über den Abstand des Gegenstandes von der Vorrichtung am jeweiligen Leuchtfleck des Punktrasters bestimmt werden kann. Von Vorteil ist es ferner, wenn eine Bewegungsinformation in Tiefenrichtung auf Grundlage der Phasendifferenzen der multiplen Wellenlängen des Rasterlichtes und des Referenzlichtes ermittelbar ist.

Das Objektlicht ist oder umfasst vorzugsweise Licht des sichtbaren Spektrums, insbesondere eines zusammenhängenden Spektralbereichs. Beispielsweise ist das Objektlicht Umgebungslicht. Alternativ kann zum Beispiel quasi-monochromatisches Licht zum Einsatz kommen, beispielsweise mit rotem, grünem und blauem Anteil.

Vorzugsweise weisen das Rasterlicht und das Objektlicht unterschiedliche Spektren auf, um eine Separation des Objektlichts und des Rasterlichts wellenlängenabhängig zu ermöglichen und dadurch die Auswertung zu vereinfachen.

Das Objektlicht ist beispielsweise nichtkohärent, insbesondere zur Vermeidung von zur Beobachtung der Szene unerwünschten Interferenzmustern (Speckles), oder teilkohärent, zum Beispiel bei quasimonochromatischem Licht.

Günstigerweise umfasst die Beleuchtungseinheit mindestens einen Lichtleiter für Rasterlicht im Gehäuse und ein insbesondere distal am Gehäuse angeordnetes optisches Auskoppelelement. Dadurch kann beispielsweise zumindest distal der Vorrichtung eine kompakte Bauform erzielt werden. Die Lichtquelle kann demgegenüber räumlich einen großen Abstand zum Auskoppelelement aufweisen. Eine derartige Ausgestaltung eignet sich zum Beispiel für endoskopische Vorrichtungen.

Der Lichtleiter kann zum Beispiel mindestens einen Stab oder eine optische Faser umfassen und dementsprechend starr und/oder flexibel ausgestaltet sein. Denkbar ist zum Beispiel der Einsatz eines GRIN-Lichtleiters (gradient index), um eine räumliche Kollimation zu erzielen.

Der mindestens eine Lichtleiter ist beispielsweise entlang einer Gehäusewand des Gehäuses geführt, insbesondere innenseitig an der Gehäusewand. Die Gehäusewand ist zum Beispiel eine Außenwand.

Das Auskoppelelement ist oder umfasst beispielsweise ein planares Hologramm, zur Auffächerung des Rasterlichts als Punktraster auf den Gegenstand.

Die Beleuchtungseinheit umfasst vorteilhafterweise eine Lichtquelle zum Bereitstellen des Rasterlichts und des Referenzlichts, die vorzugsweise im Gehäuse angeordnet ist.

Mittels der Beleuchtungseinheit kann bei einer bevorzugten Ausführungsform der Erfindung Rasterlicht mit zeitlichem Versatz über unterschiedliche Auskoppelelemente emittierbar und diesbezügliches Referenzlicht bereitstellbar sein. Beispielsweise kann Rasterlicht jeweils getaktet über unterschiedliche Auskoppelelemente emittiert werden, um verschiedene Punktraster zu erzeugen und eine größere Fläche des Gegenstandes abzudecken. Rasterlicht kann zum Beispiel durch unterschiedliche Lichtleiter zum distalen Ende der Vorrichtung geführt und dort ausgekoppelt werden.

Die Beleuchtungseinheit umfasst vorzugsweise eine Lichtquelle zum Bereitstellen des Objektlichts, die vorzugsweise im Gehäuse angeordnet ist. Auf diese Weise kann der Gegenstand gezielt beleuchtet und dadurch das Signal-zu-Rausch-Verhältnis verbessert werden.

Vorteilhafterweise umfasst die Beleuchtungseinheit mindestens einen Lichtleiter für Objektlicht im Gehäuse und ein insbesondere distal am Gehäuse angeordnetes optisches Auskoppelelement. Wie vorstehend erwähnt kommt hierbei zum Beispiel mindestens ein Stab und/oder eine Faser zum Einsatz.

Bei einer bevorzugten Ausführungsform der Erfindung ist ein gemeinsamer Bildsensor vorgesehen, der sensitiv auf das Spektrum des Objektlichtes und auf das Spektrum des Rasterlichtes ist. Dies gibt die Möglichkeit einer konstruktiv einfachen Ausgestaltung der Vorrichtung bei zugleich kompakter Bauform. Insbesondere kann nur genau ein Bildsensor vorgesehen sein.

Bei einer andersartigen vorteilhaften Ausführungsform der Erfindung umfasst die Vorrichtung zwei Bildsensoren, wobei über ein optisches Element der optischen Einheit Objektlicht auf einen der Bildsensoren und Rasterlicht auf den anderen Bildsensor leitbar ist.

Die Bildsensoren sind vorzugsweise für das jeweils detektierte Licht sensitiver als für das jeweils andere Licht. Insbesondere ist die Quanteneffizienz eines jeweiligen Bildsensors möglichst hoch und bevorzugt maximal für das jeweils eingesetzte Licht, Objektlicht und/oder Rasterlicht.

Das vorstehend erwähnte optische Element ist oder umfasst vorzugsweise mindestens ein wellenlängensensitives Strahlteilerelement, insbesondere einen Strahlteilerwürfel oder eine Strahlteilerplatte.

Die optische Einheit umfasst günstigerweise mindestens ein polarisierendes Element, insbesondere für das Rasterlicht und/oder das Referenzlicht. Beispielsweise umfasst der Strahlteilerwürfel oder die Strahlteilerplatte eine polarisierende Schicht, um unerwünschte Signalbeiträge zu verringern und das Signal-zu-Rausch-Verhältnis zu verbessern.

Die optische Einheit umfasst bevorzugt mindestens ein Filterelement zur Streulichtreduktion, das vorzugsweise eintrittsseitig angeordnet ist.

Die optische Einheit kann vorteilhafterweise eintrittsseitig ein lichtbrechendes optisches Element für das Objektlicht umfassen. Das lichtbrechende optische Element ist beispielsweise eine Linse, zum Beispiel eine Sammellinse oder ein Mikrolinsenarray. Das Mikrolinsenarray kann beispielsweise konvex gekrümmt sein.

Zwei Bildsensoren können vorgesehen sein, die bei einer bevorzugten Ausführungsform der Erfindung seitlich nebeneinander positioniert sind, insbesondere in einer gemeinsamen Ebene, und von mindestens einem Eintrittsfenster überdeckt. Beispielsweise kommt ein gemeinsames Eintrittsfenster oberhalb der Bildsensoren zum Einsatz. Durch die Positionierung der Bildsensoren seitlich nebeneinander kann vorzugsweise eine flache Bauform erzielt werden.

Dementsprechend ist die Vorrichtung vorzugsweise in Flachbauweise ausgestaltet, und das Eintrittsfenster an einer Oberfläche des Gehäuses angeordnet, entlang der die Erstreckung der Vorrichtung wesentlich größer ist als in einer Richtung quer dazu.

Bei einer bevorzugten Ausführungsform der Erfindung können zwei Bildsensoren vorgesehen sein, die in im Winkel zueinander ausgerichteten Ebenen angeordnet sind, und dass ein wellenlängensensitives Strahlteilerelement einem jeweiligen Bildsensor in Einfallsrichtung des Objektlichtes bzw. des Rasterlichtes vorgelagert angeordnet ist. Dies ermöglicht vorzugsweise eine in drei Dimensionen kompakte Bauform der Vorrichtung. Das Strahlteilerelement ist zum Beispiel ein Strahlteilerwürfel oder eine Strahlteilerplatte und transmittiv für das eine Licht (beispielsweise das Rasterlicht) und reflektiv für das andere Licht (beispielsweise das Objektlicht). Vorzugsweise grenzen die Bildsensoren unmittelbar an das Strahlteilerelement oder sind diesem nahe angeordnet. Der Winkel kann vorzugsweise 90° oder im Wesentlichen 90° betragen.

Die optische Einheit umfasst vorteilhafterweise einen für Objektlicht sensitiven Bildsensor in Einfallrichtung des Objektlichts vorgelagert ein phasen- und/oder amplitudenmodulierendes optisches Element. Hierdurch können beispielsweise auf dem Bildsensor überlagerte Bilder erzeugt werden. Dabei kann zum Beispiel ein Mikrolinsenarray für Multifokal-Abbildungen eingesetzt werden. Das optische Element kann alternativ zum Beispiel eine Phasenmaske oder eine Amplitudenmaske sein (US 8,243,353 B1).

Der mindestens eine Bildsensor, insbesondere die Bildsensoren, und/oder die optische Einheit sind vorteilhafterweise an einem distalen Ende oder Endabschnitt der Vorrichtung angeordnet, um eine kompakte Bauform zu erzielen.

Als günstig erweist es sich, wenn das Referenzlicht mit planarer oder im Wesentlichen planarer Wellenfront auf den mindestens einen Bildsensor einstrahlt.

Die Wellenfront ist relativ zu einer Ebene des mindestens einen Bildsensors vorzugsweise geneigt, im Hinblick auf eine verbesserte Auswertung. Vorgesehen ist insbesondere, dass die Neigung von einer Wellenlänge des Referenzlichts abhängt. Die Wellenfronten unterschiedlicher Wellenlängen weisen dementsprechend vorzugsweise unterschiedliche Neigungen relativ zur Ebene auf.

Die optische Einheit umfasst vorzugsweise ein optisches Element zum Auskoppeln von Referenzlicht gehäuseintern. Beispielsweise wird Licht der Lichtquelle in Rasterlicht und in Referenzlicht unterteilt, das über mindestens einen Lichtleiter im Gehäuse ausgekoppelt wird. Eine Emission von Referenzlicht extern und anschließende Einkopplung in die Vorrichtung kann unterbleiben. Dies begünstig den kompakten Aufbau der Vorrichtung.

Die optische Einheit umfasst vorzugsweise ein VPH (Volume-Phase-Hologram) zur Beugung des Referenzlichts in Richtung des mindestens einen Bildsensors. Über das VPH können beispielsweise die vorstehend erwähnten planaren Wellenfronten unterschiedlicher Wellenlängen des Referenzlichtes gebeugt werden.

Das VPH kann insbesondere ein Transmissions- oder Reflexionsgitter sein oder umfassen. Bei Erfüllung der Bragg-Bedingung kann Referenzlicht am Gitter in Richtung des Bildsensors gebeugt werden.

Das VPH ist bei einer bevorzugten Ausführungsform der Erfindung an einer Eintrittsseite der Vorrichtung positioniert und transmittiv für das Objektlicht und/oder das Rasterlicht. Beispielsweise wird das Referenzlicht durch das VPH in Richtung des Bildsensors zurückgebeugt und interferiert dort mit dem Rasterlicht.

Alternativ oder ergänzend kann vorgesehen sein, dass das VPH parallel zu einer Ebene eines Bildsensors ausgerichtet ist und dem Bildsensor in Einfallsrichtung des Rasterlichts unmittelbar vorgelagert angeordnet ist.

Bei einer bevorzugten Ausführungsform der Erfindung umfasst die Beleuchtungseinheit eine Mehrzahl von nebeneinander in einer Reihe angeordneten Mikrolinsen, die eine im Wesentlichen planare Wellenfront des Referenzlichtes oberhalb einer Ebene des mindestens einen Bildsensors erzeugen. Dies bietet sich beispielsweise für eine Flachbauweise der Vorrichtung an. Alternativ können zum Beispiel ein Multi-Volumen-Hologramm oder ein Array aus GRIN-Linsen vorgesehen sein.

Die optische Einheit umfasst günstigerweise ein optisches Element zur Aufweitung von Referenzlicht mit planarer oder im Wesentlichen planarer Wellenfront in Richtung des mindestens einen Bildsensors, wobei das optische Element zum Beispiel ein Hohlspiegel ist. Das optische Element kann insbesondere anstelle des VPHs zum Einsatz kommen

Die erfindungsgemäße Vorrichtung umfasst vorteilhafterweise einen kompakten Aufbau.

Unter der Annahme mindestens eines Bildsensors mit quadratischer Querschnittsfläche der Fläche a², wobei a eine Kantenlänge ist, weist die Vorrichtung eintrittsseitig vorteilhafterweise eine Querschnittsfläche von weniger als 1,5a² auf, vorzugsweise weniger als 1,25a². Etwaige, über die Fläche des Bildsensors hinausragende Komponenten der Vorrichtung (beispielsweise der optischen Einheit und des Gehäuses) können besonders kompakt ausgestaltet werden.

Kommt ein Strahlteilerelement wie zum Beispiel ein Strahlteilerwürfel zum Einsatz, kann dessen Abmessung ungefähr derjenigen der Bildsensoren entsprechen. Die optischen Komponenten der optischen Einheit können dadurch vorzugsweise in einem Volumen von ungefähr a³ untergebracht werden.

Unter der Annahme mindestens eines Bildsensors mit quadratischer Querschnittsfläche der Fläche a², wobei a eine Kantenlänge ist, weist die Vorrichtung bei Vorhandensein eines Bildsensors oder von zwei Bildsensoren in Flachbauweise bevorzugt eine Aufbauhöhe von ungefähr a/4 oder weniger auf und ein Volumen eintrittsseitig von a³/2 oder weniger.

Eine Abtastrate zur Aufnahme der Bilddatensätze beträgt vorzugsweise ungefähr 250 kHz oder mehr, vorzugsweise bis ungefähr 1 kHz oder mehr.

Eine jeweilige Spotgröße des Rasterlichts auf dem Gegenstand beträgt vorzugsweise ungefähr 50 µm bis 500 µm, bevorzugt ungefähr 100 µm bis 250 µm.

Ein Arbeitsabstand der Vorrichtung vom Gegenstand kann unterschiedlich ausfallen und beispielsweise von wenigen mm bis zu einigen Hundert mm betragen. Beispielsweise beträgt der Arbeitsabstand ungefähr 50 mm bis 100 mm.

Die Rasterpunkte des Rasterlichtes können auf dem Gegenstand vorzugsweise einen Abstand von jeweils ungefähr 1 mm voneinander aufweisen, wobei der Abstand von dem Arbeitsabstand der Vorrichtung zum Gegenstand abhängen kann.

Wie bereits erwähnt ist die Vorrichtung vorzugsweise handhaltbar und/oder handführbar.

Die Vorrichtung kann insbesondere eine endoskopische Vorrichtung sein, die zumindest abschnittsweise mit dem Gehäuse in den Untersuchungsgegenstand einbringbar ist. Der Untersuchungsgegenstand kann ein menschlicher oder tierischer Körper sein oder eine Sache. Eine Anwendung im Dentalbereich kann vorgesehen sein, wobei die Vorrichtung ganz oder teilweise in die Mundhöhle einbringbar ist.

Die Vorrichtung kann beispielsweise in der industriellen Messtechnik eingesetzt werden.

Die Vorrichtung ist vorzugsweise ein tragbares Kommunikationsgerät oder ist von einem solchen umfasst, insbesondere ein Smartphone oder ein Tablet-Computer.

Bei einer beispielhaften Ausführungsform ist die Vorrichtung ein Head-mounted-Gerät, zum Beispiel eine Datenbrille, speziell für AR-Anwendungen (AR, Augmented Reality).

Dementsprechend bezieht sich die vorliegende Erfindung auch auf eine endoskopische Vorrichtung, ein Smartphone oder ein Tablet-Computer und/oder eine Head-mounted-Gerät, umfassend mindestens eine Vorrichtung der vorstehend beschriebenen Art.

Die eingangs genannte Aufgabe wird durch ein erfindungsgemäßes Verfahren mit den Merkmalen des unabhängigen Anspruches 25 gelöst.

Die bereits im Zusammenhang mit der Erläuterung der erfindungsgemäßen Vorrichtung erwähnten Vorteile können unter Einsatz des Verfahrens ebenfalls erzielt werden. Die Vorrichtungsmerkmale können verfahrensgemäß umgesetzt sein. Vorteilhafte Ausführungsbeispiele des Verfahrens ergeben sich durch vorteilhafte Ausführungsformen der Vorrichtung. Diesbezüglich kann auf die voranstehenden Ausführungen verwiesen werden.

Die nachfolgende Beschreibung bevorzugter Ausführungsformen der Erfindung, bei denen es sich insbesondere um 3D-Scanner handelt, dient im Zusammenhang mit der Zeichnung der näheren Erläuterung der Erfindung. Es zeigen:
- Figur 1:: eine schematische perspektivische Darstellung einer erfindungsgemäßen Vorrichtung, ausgestaltet als endoskopische Vorrichtung, sowie einen Benutzer;
- Figur 2:: eine schematische Darstellung eines distalen Endabschnittes der Vorrichtung aus Figur 1 sowie einen zu untersuchenden Gegenstand;
- Figur 3:: schematisch den Gegenstand mit einem Punktraster von Rasterlicht;
- Figur 4:: eine schematische Darstellung eines Leuchtflecks des Referenzlichts auf dem Gegenstand mit Specklemuster;
- Figur 5:: einen Leuchtfleck mit Specklemuster zu aufeinanderfolgenden Zeitpunkten, wobei ein überlappender Bereich identischer Specklemuster durch einen Rahmen schematisch hervorgehoben ist und Pfeil eine Bewegung des Leuchtflecks kennzeichnet;
- Figur 6:: drei beispielhafte Bilddatensätze zu aufeinanderfolgenden Zeitpunkten und, schraffiert, einen in diesen enthaltenen überlappenden Bereich, wobei Pfeile eine Verschiebung von zwei Bilddatensätzen abhängig von einer Bewegungsinformation zurück auf den ersten Bilddatensatz symbolisch darstellen;
- Figur 7:: einen distalen Abschnitt einer weiteren bevorzugten Ausführungsform der erfindungsgemäßen Vorrichtung in schematischer Darstellung;
- Figur 8:: eine schematische Darstellung einer weiteren bevorzugten Ausführungsform der erfindungsgemäßen Vorrichtung;
- Figur 9:: eine schematische Ansicht der Vorrichtung aus Figur 8 in Blickrichtung des Pfeiles "9";
- Figur 10:: eine schematische Teildarstellung einer weiteren bevorzugten Ausführungsform der erfindungsgemäßen Vorrichtung;
- Figuren 11 und 12:: schematische Darstellungen bevorzugter Ausführungsformen der Erfindung.

Mit den nachfolgend erläuterten vorteilhaften Ausführungsformen der erfindungsgemäßen Vorrichtung kann ein bevorzugtes Ausführungsbeispiel des erfindungsgemäßen Verfahrens ausgeübt werden.

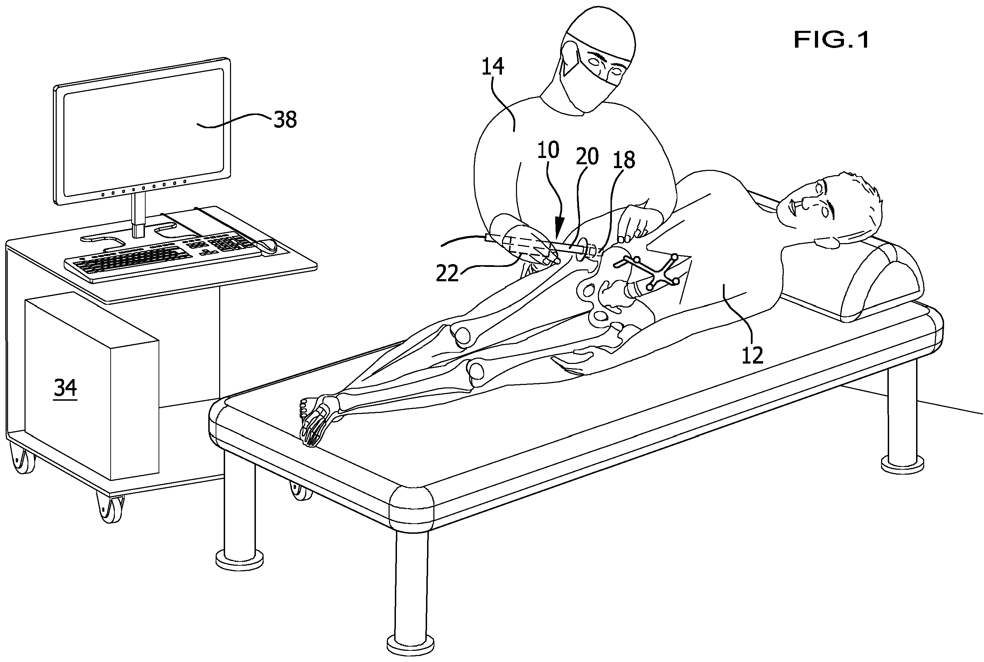

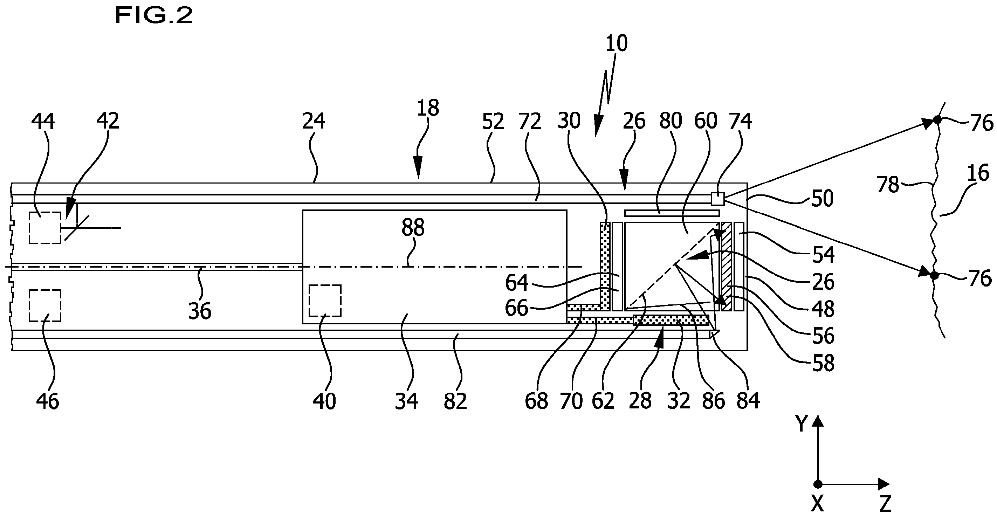

Die Erfindung wird nachfolgend zunächst am Beispiel der Figuren 1 bis 5 und der Ausführungsform der Erfindung in den Figuren 1 und 2 erläutert. Die in diesem Zusammenhang erwähnten Vorteile gelten auch für die anschließend erläuterten bevorzugten Ausführungsformen der Erfindung, so dass diesbezüglich auf die nachfolgend zunächst gemachten Ausführungen verwiesen werden kann. Es werden lediglich die wesentlichen Unterschiede zwischen den unterschiedlichen Ausführungsformen erläutert.

Für gleiche oder gleichwirkende Merkmale und Bauteile werden identische Bezugszeichen benutzt.

Figur 1 zeigt in schematischer Darstellung eine mit dem Bezugszeichen 10 bevorzugte Ausführungsform der erfindungsgemäßen Vorrichtung. Die Vorrichtung 10 ist insbesondere eine endoskopische Vorrichtung, die im vorliegenden Beispiel zur Untersuchung eines Patienten 12 eingesetzt wird. Die Vorrichtung 10 ist handhaltbar und handgeführt und wird von einem Benutzer 14 bedient.

Es versteht sich, dass die Vorrichtung 10 nur beispielhaft bei einer Anwendung in der Medizintechnik dargestellt ist. Die Erfindung kann endoskopisch auch zur Untersuchung von nichtlebenden Gegenständen eingesetzt werden. Ferner ist hervorzuheben, dass die Vorrichtung 10 selbst bei Einsatz im medizinischen Umfeld lediglich zu Informationszwecken anstelle von Diagnosezwecken eingesetzt werden kann.

Im vorliegenden Fall ist die Vorrichtung 10 durch eine Körperöffnung ins Körperinnere des Patienten 12 geführt, um einen Gegenstand 16 optisch zu untersuchen (Figur 2). Hierbei soll ein 3D-Oberflächendatensatz des Gegenstandes 16 erstellt werden.

Die Vorrichtung 10 weist einen distalen Abschnitt 18 auf, der ins Körperinnere eingeführt ist, und einen proximalen Abschnitt 20. Am proximalen Abschnitt 20 ist beispielsweise ein Griffelement 22 für den Benutzer 14 angeordnet.

Der distale Abschnitt 18 umfasst im vorliegenden Ausführungsbeispiel ein insbesondere schaftförmiges Gehäuse 24 zur Aufnahme von Komponenten der Vorrichtung 10. Es besteht insbesondere der Vorteil einer hohen baulichen Integration zur Erzielung eines kompakten Aufbaus, bei dem zahlreiche Komponenten im Gehäuse 24 angeordnet sind. Insbesondere kann die Mehrzahl der Komponenten am distalen Abschnitt 18 angeordnet sein.

Die Vorrichtung 10 weist eine im Gehäuse 24 angeordnete optische Einheit 26 auf, eine im Gehäuse 24 angeordnete Sensoreinheit 28 mit zwei Bildsensoren 30, 32 und ferner mindestens eine Datenverarbeitungseinheit 34. Im vorliegenden Beispiel ist die Datenverarbeitungseinheit 34 im Gehäuse 24 angeordnet und dargestellt.

Bildsignale der Bildsensoren 30, 32 können der Datenverarbeitungseinheit 34 zugeführt und von dieser verarbeitet werden. Zu diesem Zweck ist die Datenverarbeitungseinheit 34 ausgebildet und programmiert, entsprechende Berechnungen vorzunehmen. Hierfür kann ein Anwendungsprogramm ausführbar in der Datenverarbeitungseinheit 34 oder einer mit dieser verbundenen Speichereinheit gespeichert sein.

Eine Datenverarbeitungseinheit 34, die Bestandteil der Vorrichtung 10 sein kann oder nicht, kann alternativ außerhalb des Gehäuses 24 positioniert sein (Figur 1). Vorgesehen sein kann beispielsweise, dass in der Datenverarbeitungseinheit 34 gehäuseintern Informationen nur teilweise verarbeitet oder vorverarbeitet werden und an die weitere Datenverarbeitungseinheit 34 außerhalb des Gehäuses übertagen werden, wobei diese Datenverarbeitungseinheit 34 eine weitergehende Auswertung oder Detailauswertung vornimmt. Eine Leitung 36 kann zur Datenübertragung vorgesehen sein und ist vorzugsweise eine Hochgeschwindigkeits-Datenleitung.

Von der mindestens einen Datenverarbeitungseinheit 34 kann beispielsweise eine Anzeigeeinheit 38 zur Darstellung der Szene angesteuert werden. Die Anzeigeeinheit 38 kann Bestandteil der Vorrichtung sein. Darüber hinaus kann eine Speichereinheit 40 zur Speicherung von Datensätzen der Vorrichtung 10 vorgesehen sein, die separat von der Datenverarbeitungseinheit 34 gebildet oder in diese integriert ist.

Datenübertragungen zwischen Komponenten der Vorrichtung 10 und/oder eine Datenübertragung mit externen Komponenten, beispielsweise der Anzeigeeinheit 38 oder einer Auswerteeinheit, kann kabellos und/oder kabelgebunden sein.

Die Vorrichtung 10 umfasst ferner eine Beleuchtungseinheit 42 zur Bereitstellung von Licht, wie nachfolgend erläutert insbesondere von Rasterlicht und Referenzlicht. Zu diesem Zweck umfasst die Beleuchtungseinheit 42 eine Lichtquelle 44, die vorzugsweise im Gehäuse 24 und speziell im distalen Abschnitt 18 angeordnet sein kann. Alternativ kann die Lichtquelle 44 im proximalen Abschnitt 20 angeordnet sein oder gar separat, wobei ein Lichtleiter zur Zuführung des Lichts vorgesehen sein kann.

Die Lichtquelle 44 ist vorliegend eine Laserlichtquelle. Über ein Strahlteilerelement können Rasterlicht und Referenzlicht getrennt und in den jeweiligen optischen Pfad eingekoppelt werden.

Des Weiteren kann die Beleuchtungseinheit 42 eine Lichtquelle 46 umfassen zum Bereitstellen von Objektlicht. Die Lichtquelle 46 ist bei der Vorrichtung 10 gemäß Figur 2 nicht umgesetzt und daher nur schematisch dargestellt. Sie könnte jedoch wie bei den nachfolgend erläuterten Ausführungsformen ebenfalls vorhanden sein.

An einer distalen Stirnseite des Gehäuses 24 sind eine Eintrittsöffnung 48 und eine Austrittsöffnung gebildet, wobei die Eintrittsöffnung 48 vorzugsweise zentral und die Austrittsöffnung 50 im vorliegenden Beispiel an einer Außenwand 52 angeordnet ist.

Die optische Einheit 26 umfasst in Einfallsrichtung des von außen stammenden Lichtes ein Filterelement 54, speziell ein IR-Filter, und daran anschließend ein Volume-Phase-Hologram (VPH) 56. Das VPH 56 ist beispielsweise als transmittives Beugungsgitter 58 ausgestaltet. Das VPH 56 kann unter der Bragg-Bedingung reflektieren und wie nachfolgend erläutert als Hohlspiegel für das Referenzlicht wirken.

In Richtung einfallenden Lichts folgt auf das VPH 56 ein Strahlteilerelement, ausgestaltet als Strahlteilerwürfel 60. Der Strahlteilerwürfel 60 umfasst eine Reflexionsschicht 62 zur Reflexion des Rasterlichts. Demgegenüber wird Licht des sichtbaren Spektrums von der Reflexionsschicht 62 transmittiert.

Anstelle des Strahlteilerwürfels 60 kann ein andersartiges wellenlängensensitives Strahlteilerelement zum Einsatz kommen, zum Beispiel eine Strahlteilerplatte.

Sichtbares Licht durchläuft den Strahlteilerwürfel 60 und kann auf den Bildsensor 30 gelangen. In Richtung des einfallenden Lichts dem Bildsensor 30 vorgelagert umfasst die optische Einheit 26 ein optisches Element 64, das phasen- und/oder amplitudenmodulierend ist. Im vorliegenden Beispiel ist das optische Element 64 ein Mikrolinsenarray 66. Über das Mikrolinsenarray 66 wird die beobachtete Szene auf den Bildsensor 30 abgebildet.

Der Bildsensor 30 steht über eine Signalleitung 68 mit der Datenverarbeitungseinheit 34 in Wirkverbindung.

Das vom Strahlteilerwürfel 60 reflektierte NIR-Licht gelangt auf den Bildsensor 32. Der Bildsensor 32 steht über eine Signalleitung 70 mit der Datenverarbeitungseinheit 34 in Wirkverbindung.

Die Beleuchtungseinheit 42 umfasst einen Lichtleiter 72 zum Zuführen von Rasterlicht von der Lichtquelle 44. Der Lichtleiter 72 kann zum Beispiel einen Stab und/oder eine Faser umfassen und starr oder flexibel ausgestaltet sein.

Das Rasterlicht wird zu einem optischen Auskoppelelement 74 an der Austrittsöffnung 50 geführt. Das Auskoppelelement 74 ist beispielsweise ein planares Hologramm zum Erzeugen eines Musters von Leuchtflecken 76 (Spots) auf der Oberfläche des Gegenstandes 16.

Anstelle der Nutzung eines planaren Hologramms als Auskoppelelement 74 kann zum Beispiel ein räumlicher Lichtmodulator (SLM, spatial light modulator) vorgesehen sein. Der Modulator kann vorzugsweise im distalen Abschnitt 18 oder auch im proximalen Abschnitt 20 angeordnet sein. Beispielsweise kommt der Modulator zum Einsatz, wenn ein Tiefenbereich der Vorrichtung 10 begrenzt ist und pro Leuchtfleck 76 eine erhöhte Lichtintensität erforderlich ist.

Das Referenzlicht umfasst im vorliegenden Fall Licht des NIR und/oder sichtbaren Wellenlängenbereiches von ungefähr 700 nm oder weniger bis 1300 nm. Vorzugsweise umfasst das Spektrum eine Mehrzahl von diskreten Wellenlängen in einem Wellenlängenfenster von ungefähr 1 nm bis 10 nm Breite. Der Wellenlängenabstand beträgt beispielsweise ungefähr 1nm.

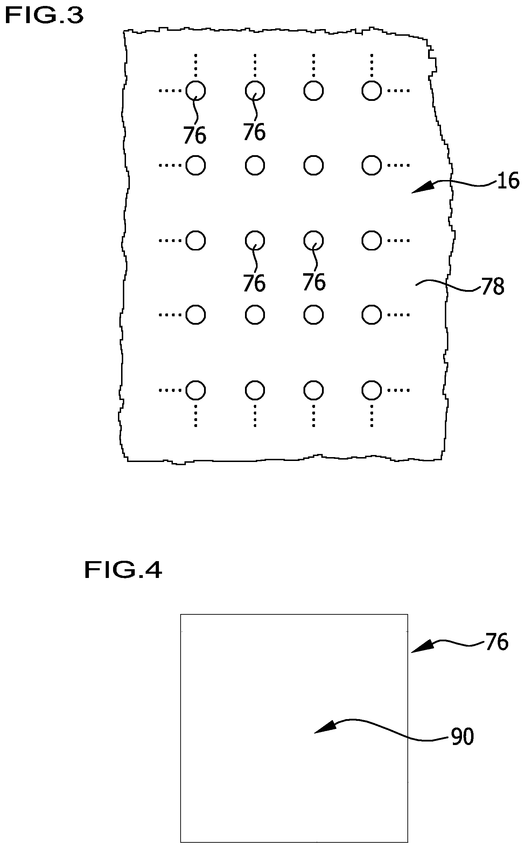

Figur 3 stellt schematisch die Leuchtflecken 76 des Punktrasters auf der Oberfläche 78 des Gegenstandes 16 dar. Ein jeweiliger Leuchtfleck 76 weist beispielsweise eine Größe von ungefähr 100 µm auf. Die Rastergröße beträgt beispielsweise ungefähr 1 mm und kann wie erwähnt vom Arbeitsabstand abhängen.

Das von der Oberfläche 78 reflektierte Rasterlicht durchquert das Filterelement 54 und das VPH 56. An der Reflexionsschicht 62 wird das Rasterlicht in Richtung des Bildsensors 32 reflektiert. Vorgesehen sein kann, dass die Reflexionsschicht 104 polarisierend ist und/oder dass die optische Einheit 26 ein polarisierendes optisches Element umfasst.

Der Bildsensor 32 ist hinsichtlich seiner Sensitivität auf Licht im Spektralbereich des Rasterlichts optimiert. Etwaige Rückreflexionen von der Oberfläche des Bildsensors 32, die durch die Reflexionsschicht 62 hindurchgelangen, können von einem Absorberelement 80 an der dem Bildsensor 32 gegenüberliegenden Seite absorbiert werden.

Zusätzlich zum Rasterlicht kommt gehäuseinternes Referenzlicht der Lichtquelle 44 zum Einsatz. Im vorliegenden Beispiel wird das Referenzlicht nach dem bereits erwähnten Strahlteilerelement (zum Beispiel einen Faserkoppler) über einen Lichtleiter 82 in Richtung des distalen Endes des Gehäuses 24 geführt. Der Lichtleiter 82 kann als Stab oder Faser, starr oder flexibel ausgestaltet sein. Endseitig umfasst der Lichtleiter 82 ein optisches Auskoppelelement 84, vorliegend ausgestaltet als Reflektor und insbesondere Prismenreflektor.

Vom Auskoppelelement 84 wird Referenzlicht vorzugsweise mit sphärischer Wellenfront in Richtung der Reflexionsschicht 62 emittiert. Von dieser wird Referenzlicht in das VPH 56 reflektiert.

Im vorliegenden Fall ist die jeweilige Bragg-Bedingung für Referenzlicht im VPH 56 erfüllt. Referenzlicht wird vom VPH 56 zurückgebeugt, wobei eine im Wesentlichen planare Wellenfront 86 entsteht. Hierbei ist das System aus auswertungstechnischen Gründen vorteilhafterweise so ausgelegt, dass die Wellenfront relativ zu einer von der Oberfläche des Bildsensors 32 definierten Ebene geneigt ist.

Die Wellenfronten 86 (nur eine gezeigt) der verschiedenen Wellenlängen im Referenzlicht weisen aufgrund der jeweiligen Bragg-Bedingung voneinander eine unterschiedliche Neigung auf.

Im Bildsensor 32 interferiert das Rasterlicht mit dem Referenzlicht. Die diesbezüglichen Bildsignale werden an die Datenverarbeitungseinheit 34 übertragen.

Die Datenverarbeitungseinheit 34 kann auf vorstehend beschriebene Weise mittels digitaler optischer Holografie ein dreidimensionales Punktraster aus den interferierenden Signalen des Rasterlichts und Referenzlichts erstellen. Ein diesbezüglicher Referenzdatensatz ist indikativ für eine Lateralinformation und eine Tiefeninformation und wird von der Datenverarbeitungseinheit 34 bereitgestellt. "Lateral" ist vorliegend insbesondere als quer und vorzugsweise senkrecht zu einer von der Vorrichtung 10 definierten Achse 88 bezogen aufzufassen.

Über das Punktraster werden Stützstellen für die nachfolgend erläuterte Registrierung des Bilddatensatzes der Oberfläche 78 bereitgestellt. In gewisser Weise kann das Punktraster die Oberfläche 78 an den Punkten als Hüllfläche des Gegenstandes 16 abtasten.

Vorgesehen sein kann, dass die Lichtleiter 72, 82 in Querrichtung der Vorrichtung 10, vorliegend senkrecht zur Zeichenebene, ungefähr mittig zum Strahlteilerwürfel 60 angeordnet sind, um die Ausbreitung der sphärischen Welle in Richtung des VPH 56 zu erleichtern.

Der Referenzdatensatz umfasst die Lateralinformation über die Leuchtflecken 76 sowie darüber hinaus eine absolute Tiefeninformation. Die Tiefeninformation ergibt sich durch die Phasendifferenzen der Mehrzahl der verwendeten Wellenlängen im Rasterlicht und Referenzlicht.

Das auf den Bildsensor 30 gelangende Objektlicht des Gegenstandes 16 wird in Bildsignale überführt und der mindestens einen Datenverarbeitungseinheit 34 zugeführt. Die mindestens eine Datenverarbeitungseinheit 34 erstellt einen Bilddatensatz 35 (Figur 3). Der Bilddatensatz 35 kann von der Datenverarbeitungseinheit 34 räumlich in Bezug auf den Referenzdatensatz registriert und dadurch ein 3D-Oberflächendatensatz der Oberfläche 78 bereitgestellt werden.

Bilddatensätze 35, die mit dem Objektlicht gewonnen werden, sind aufgrund von Rauschen mit Unschärfen behaftet. Aus diesem Grund kann in der Praxis beispielsweise eine ansonsten mögliche maximale Auflösung des Bilddatensatzes 35 mit der Vorrichtung 10 nicht erzielt werden. Eine typische, maximale Auflösung kann vorliegend beispielsweise im Bereich von ungefähr 25 µm liegen.

Zur Verbesserung der Bilddatensätze 35 und insbesondere zum Bereitstellen eines hochaufgelösten 3D-Oberflächendatensatzes besteht bei der Vorrichtung 10 die Möglichkeit, den Referenzdatensatz mit den Bilddatensätzen 35 wie nachfolgend erläutert in Beziehung zu setzen, um die Bildqualität zu verbessern. Zu diesem Zweck wird eine Relativbewegung der Vorrichtung 10 und des Gegenstandes 16 herangezogen, wobei diese Relativbewegung über die Vorrichtung 10 selbst ermittelt werden kann. Eine gesonderte Vorrichtung zur Feststellung der Relativbewegung ist nicht erforderlich.

Als vorteilhaft erweist es sich dabei, dass das in der Praxis zwangsläufig auftretende Zittern der handgehaltenen und handgeführten Vorrichtung 10 selbst für die Ermittlung der Relativbewegung herangezogen wird.

Beim Auftreffen des kohärenten Rasterlichts auf der Oberfläche 78 entsteht der Leuchtfleck 76. Infolge der rauen Oberflächenbeschaffenheit weist der Leuchtfleck 76 ein Specklemuster 90 auf, das in Figur 4 abschnittsweise schematisch als Intensitätsbild dargestellt ist. Hierbei symbolisieren zum Beispiel dunkle Abschnitte hohe Intensität und helle Abschnitte niedrige Intensität.

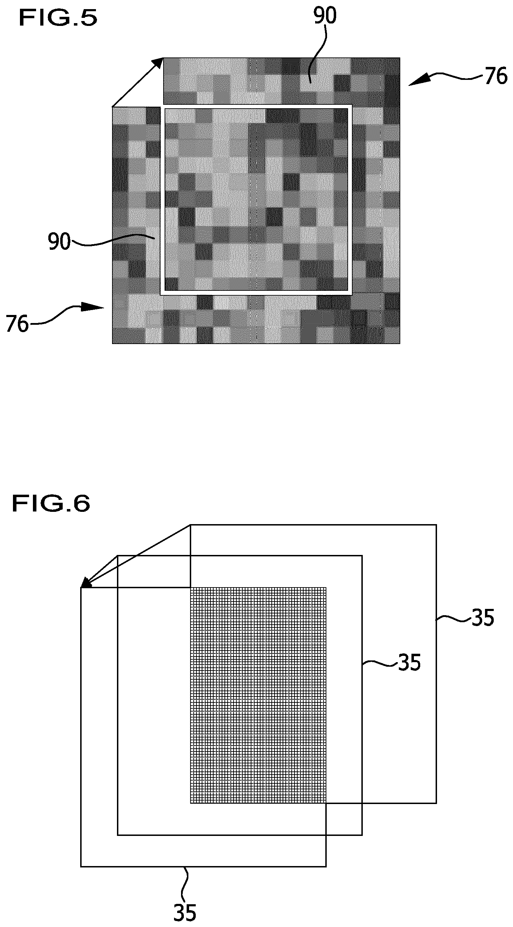

Bei einer Relativbewegung der Vorrichtung 10 und des Gegenstandes 16 wird der Leuchtfleck 76 über die Oberfläche 78 bewegt. Das Specklemuster 90 bleibt dabei ortsfest aufgrund der Beschaffenheit der Oberfläche 78 erhalten, innerhalb des Leuchtflecks 76 ist es jedoch verschoben. Dies ist in Figur 5 angedeutet, wobei ein weißer Rahmen den Überlapp schematisch einfasst.

Das Specklemuster 90 kann zeitabhängig untersucht werden. Durch diese Speckle-Korrelation aufeinanderfolgender Specklemuster 90 kann die Datenverarbeitungseinheit 34 eine Bewegungsinformation in lateraler Richtung mit hoher Genauigkeit erstellen, die indikativ für die Relativbewegung ist.

Eine Relativbewegung in Tiefenrichtung kann durch die Möglichkeit, eine absolute Tiefeninformation aufgrund der Nutzung der mehreren Wellenlängen im Rasterlicht und Referenzlicht ermittelt werden.

Im Ergebnis kann die Datenverarbeitungseinheit 34 eine Bewegungsinformation in Lateral- und Tiefenrichtung erstellen.

Die Bilddatensätze 35, die mittels des Objektlichts gewonnen werden, können anhand der Bewegungsinformation so ausgewertet werden, dass einander überlappende Bereiche zugeordnet werden können. Dies ist in Figur 6 schematisch dargestellt, die drei Bilddatensätze 35 und einen überlappenden Bereich zeigt.

Da durch die Relativbewegung der Vorrichtung 10 und des Gegenstandes 16 auch die Bildinformation in den Bilddatensätzen 35 verschoben wird, kann die inverse Bewegung auf einen Bilddatensatz 35 angewandt werden, um diesen räumlich in Übereinstimmung mit einem vorangegangenen Bilddatensatz 35 zu bringen. Auf diese Weise können die einander überlappenden Bereiche der Bilddatensätze innerhalb der Szene aufgefunden werden.

Durch rechnerische Integration der zwei oder mehr aufeinanderfolgenden Bilddatensätze kann das Signal-zu-Rausch-Verhältnis in den Bilddatensätzen verbessert werden, da das in dem jeweiligen Bilddatensatz 35 enthaltene Rauschen sich mit der Zeit ändert. Als Integration kann wie vorstehend erwähnt auch eine Mittelung angesehen werden.

Es versteht sich, dass eine hohe Framerate vorteilhaft ist. Beispielsweise können bevorzugt Frameraten von 250 Hz oder mehr eingesetzt werden, vorzugsweise mehr als 1 kHz. Die Framerate sollte so hoch wählbar sein, dass innerhalb aufeinanderfolgender Bilddatensätze möglichst große überlappende Bereiche identifiziert werden können.

Insbesondere ist es von Vorteil, wenn mehr als nur zwei Bilddatensätze miteinander geglättet werden können.

Im Ergebnis kann die Datenverarbeitungseinheit 34 einen 3D-Oberflächendatensatz des Gegenstandes 16 ohne Verlust an Detailtreue und unter Beibehaltung höherer Raumfrequenzen bereitstellen. Der 3D-Oberflächendatensatz ist hochaufgelöst und vorzugsweise bis zur Auflösungsgrenze des Systems aufgelöst.

Bei rauschfreiem oder im Wesentlichen rauschfreiem errechneten Bilddatensatz 35 oder 3D-Oberflächendatensatz besteht insbesondere die Möglichkeit, die Auflösung durch Anwendung von Entfaltungsoperationen rechnerisch weiter zu erhöhen. Alternativ oder ergänzend können zum Beispiel durch Interpolation "Superresolution"-Datensätze erstellt werden.

Wie bereits erwähnt, kann die Tiefeninformation absolut gewonnen werden. Dies bietet zum Beispiel die Möglichkeit, die Bewegungsinformation spotabhängig für einzelne Leuchtflecke 76 bereitzustellen. Beispielsweise kann an den unterschiedlichen Leuchtflecken 76 eine unterschiedliche laterale Transformationsinformation vorliegen, die bei der Verrechnung der Bilddatensätze berücksichtigt werden kann. Beispielsweise lässt sich auf diese Weise die Oberfläche 78 eines bewegten, "atmenden" Gegenstandes 16 vermessen. Aus diesem Grund eignet sich die erfindungsgemäße Vorrichtung 10 speziell für den Einsatz zur Untersuchung bewegter Gegenstände 16, zum Beispiel im Körperinneren.

3D-Oberflächendatensätze können zu einer Gesamtszene zusammengesetzt werden (Stitching). Hierbei ist vorzugsweise kein oder nur minimaler Versatz zwischen den einzelnen Datensätzen vorhanden.

Unter der Annahme, dass die Bildsensoren 30, 32 jeweils im Wesentlichen quadratisch mit einer Kantenlänge a ausgestaltet sind, kann ein besonders kompakter Aufbau der Vorrichtung 10 am distalen Abschnitt 18 erzielt werden. Die optische Einheit 26 mit dem Strahlteilerwürfel 60 ist im Bereich der Bildsensoren 30, 32 ungefähr durch deren Abmessungen bestimmt. Die Eintrittsfläche der Apertur beträgt damit ungefähr a² und das Volumen ungefähr a³ (Untergrenzen). Insgesamt kann eine Abmessung quer zur Achse 88 von vorzugsweise weniger als ungefähr 1,5a² im Querschnitt und noch günstiger weniger als ungefähr 1,25a² erzielt werden.

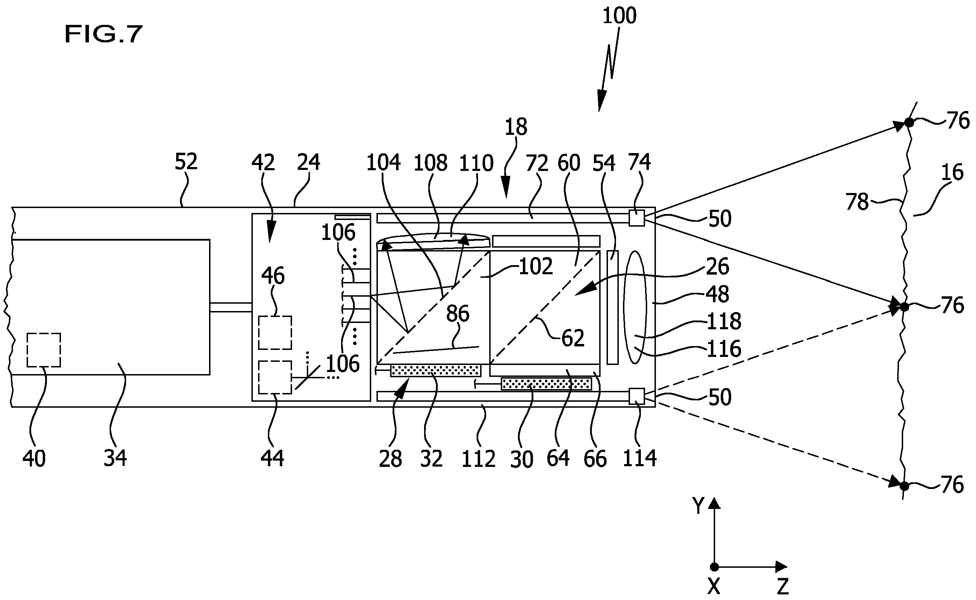

Figur 7 zeigt einen distalen Abschnitt 18 einer mit dem Bezugszeichen 100 belegten erfindungsgemäßen Vorrichtung in bevorzugter Ausführungsform. Gegenüber der Vorrichtung 10 weist die Vorrichtung 100 insbesondere den Unterschied auf, dass kein VPH 56 für das Referenzlicht zum Einsatz kommt. Stattdessen wird zusätzlich zum Strahlteilerwürfel 60 ein weiteres optisches Element in Gestalt eines Strahlteilerwürfels 102 eingesetzt.

Abweichend von der Vorrichtung 10 sind die Bildsensoren 30, 32 andersartig positioniert. Bei der Vorrichtung 10 definieren die Bildsensoren 30, 32 in einem Winkel von im Wesentlichen 90° ausgerichtete Ebenen und rahmen in gewisser Weise den Strahlteilerwürfel 60 und das Mikrolinsenarray 66 ein.

Demgegenüber sind die Ebenen der Bildsensoren 30, 32 bei der Vorrichtung 100 parallel zueinander oder fallen zusammen. Die Strahlteilerwürfel 60, 102 sind seitlich neben den Bildsensoren 30, 32 angeordnet und einem jeweiligen Bildsensor 30, 32 zugeordnet.

Bei der Vorrichtung 100 ist der Strahlengang für das Objektlicht im Verhältnis zur Vorrichtung 10 vertauscht. Das Objektlicht wird an der Reflexionsschicht 62 reflektiert, wohingegen das Rasterlicht transmittiert wird. In dem in Einfallsrichtung dem Strahlteilerwürfel 60 nachgelagerten Strahlteilerwürfel 102 wird das Rasterlicht an einer Reflexionsschicht 104 in Richtung des Bildsensors 32 reflektiert.

Referenzlicht wird über ein Auskoppelelement 106 mit im Wesentlichen sphärischer Wellenfront ausgekoppelt. Das Auskoppelelement 106 ist auf die Rückseite der Reflexionsschicht 104 gerichtet. Von der Reflexionsschicht 104 gelangt das Referenzlicht zu einem optischen Element 108, vorliegend in Gestalt eines Hohlspiegels 110. Über den Hohlspiegel 110 wird eine im Wesentlichen planare Wellenfront 86 bereitgestellt, wie im Fall der Vorrichtung 10 mittels des VPH 56.

Das Referenzlicht kann über einen optischen Wellenleiter ausgekoppelt werden, der das vorstehend erwähnte Strahlteilerelement bildet oder umfasst. Raster- und Referenzlicht werden getrennt. Das Referenzlicht wird abhängig von der Wellenlänge an den mehreren seitlich nebeneinander angeordneten Auskoppelelementen 106 ausgekoppelt. Ein optischer Wellenleiter kann vorgesehen sein, beispielweise der zuletzt genannte Wellenleiter, der die parallel nebeneinander angeordneten Auskoppelelemente 106 umfasst.

Vorgesehen sein kann, dass die Reflexionsschicht 104 polarisierend ist und/oder dass die optische Einheit 26 ein polarisierendes optisches Element umfasst. Bei polarisierender Reflexionsschicht 104 kann sämtliches Rasterlicht auf den Bildsensor 32 reflektiert werden. Im Allgemeinen kann die Polarisation des emittierten und/oder empfangenen Rasterlichts so eingestellt werden, dass unerwünschte Reflexionen aus der optischen Einheit 26 weitgehend unterdrückt werden können.

Aufgrund der Verwendung mehrerer Wellenlängen im Referenzlicht sind wie erwähnt mehrere, seitlich nebeneinander angeordnete Auskoppelelemente 106 vorgesehen (in Figur 7 angedeutet). Dadurch ergeben sich wellenlängenabhängig unterschiedlich geneigte Wellenfronten 86, wie dies bei der Vorrichtung 10 der Fall ist.

Das Referenzlicht und das Rasterlicht werden beispielsweise von einer integrierten Beleuchtungseinheit 42 bereitgestellt, in der die Lichtquelle 44 angeordnet ist.

Figur 7 zeigt bei der Vorrichtung 100 einen weiteren Lichtleiter 112 mit daran im Bereich der Austrittsöffnung 50 angeordnetem Auskoppelelement 114. Diese Komponenten entsprechen im vorliegenden Beispiel hinsichtlich Aufbau und Anordnung dem Lichtleiter 72 und dem Auskoppelelement 74 und sind vorzugsweise nahe einer Gehäusewand angeordnet, wobei die Lichtleiter 72, 112 die Lichtführungseinrichtung 26 und die Bildsensoren 32 zwischen sich aufnehmen können.

Das Auskoppelelement 114 kann beispielsweise ebenfalls ein planares Hologramm sein. Über die beiden Auskoppelelemente 74, 114 kann der Gegenstand 16 mit zwei Mustern von Leuchtflecken 76 ausgeleuchtet werden. Um die Beiträge des jeweiligen Musters trennen zu können, kann die Ausleuchtung zum Beispiel zeitversetzt aktiviert und die Aufnahme über den Bildsensor 32 entsprechend synchronisiert werden.

Objektlicht kann über die Vorrichtung 100 optional ebenfalls bereitgestellt und ausgekoppelt werden. Hierzu kommt zum Beispiel der Lichtleiter 112 zum Einsatz, ohne Auskoppelelement 114.

Die Vorrichtung 100 gemäß Figur 7 umfasst eintrittsseitig ferner ein optisches Element 116 in Gestalt einer Sammellinse 118 zur Vorfokussierung und zur Vergrößerung der Weitwinkligkeit der Vorrichtung 100.

Bei der Vorrichtung 100 kann anstelle der Sammellinse 118 zur Erhöhung der Weitwinkligkeit beispielsweise ein gekrümmtes Mikrolinsenarray eingesetzt werden, zum Beispiel in Form einer Kugelschale.

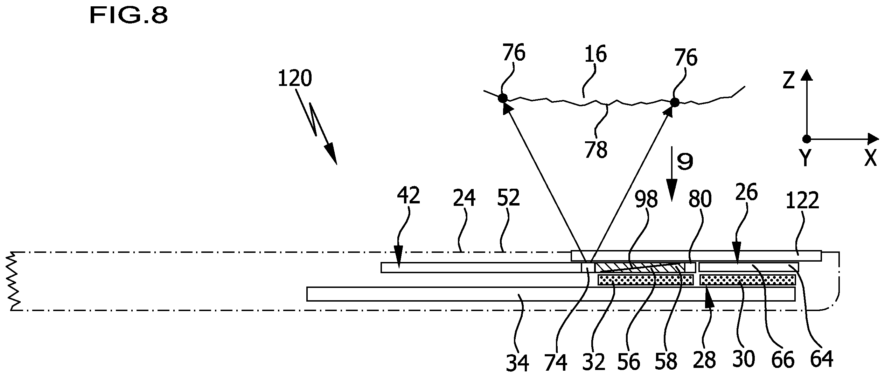

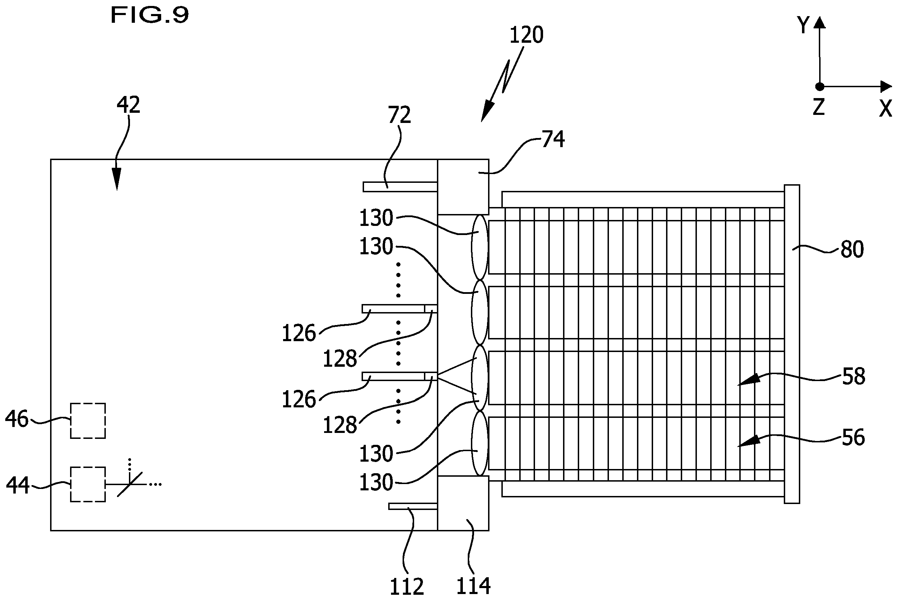

Die Figuren 8 und 9 zeigen in schematischer Darstellung eine mit dem Bezugszeichen 120 belegte vorteilhafte Ausführungsform der erfindungsgemäßen Vorrichtung. Die Vorrichtung 120 zeichnet sich insbesondere durch einen Flachaufbau aus, bei dem die Erstreckung in einer Richtung, vorliegend der Höhenrichtung in der Zeichnung, wesentlich geringer ist als in den quer dazu ausgerichteten Richtungen.

Aus diesem Grund eignet sich die Vorrichtung 120 insbesondere zum Einbau in ein tragbares elektronisches Gerät, beispielsweise ein Smartphone oder einen Tablet-Computer.

Unter der Annahme quadratischer Bildsensoren 30, 32 mit jeweiliger Kantenlänge a beträgt die Licht-Eintrittsfläche 2a². Die Aufbauhöhe kann beispielsweise zu maximal a/4 erzielt und damit das optische Volumen zu maximal ungefähr a³/2 erzielt werden (Untergrenzen). Diese Abschätzung erfolgt unter Annahme einer Kantenlänge von ungefähr 8 mm für die Bildsensoren 30, 32 und einer Aufbaudicke der Komponenten von ungefähr 1 mm bis 2 mm.

Bei der Vorrichtung 120 sind die Bildsensoren 30, 32 in einer gemeinsamen Ebene angeordnet. In Einfallsrichtung vorgelagert ist vor dem Bildsensor 30 das Mikrolinsenarray 66 positioniert. Vor dem Bildsensor 32 ist das VPH 56 angeordnet. Das VPH 56 und das Mikrolinsenarray 66 liegen vorzugsweise in einer gemeinsamen Ebene.

Eintrittsseitig ist ein Eintrittsfenster 122 vorgesehen, das das VPH 56 und das Mikrolinsenarray 66 überdeckt.

Die Beleuchtungseinheit 42 kann bevorzugt zumindest teilweise in derselben Ebene seitlich neben dem VPH 56 oder dem Mikrolinsenarray 66 angeordnet sein. Die Datenverarbeitungseinheit 34 ist beispielsweise auf der der Eintrittsseite abgewandten Seite der Bildsensoren 30, 32 angeordnet. Die Bildsensoren 30, 32 können zum Beispiel auf der Datenverarbeitungseinheit 34 integral ausgebildet sein.

Die Emission von Rasterlicht kann über das Auskoppelelement 74 erfolgen, das direkt oder indirekt an die Beleuchtungseinheit 42 angekoppelt sein kann.

Bei der Vorrichtung 120 können insbesondere die Strahlteilerwürfel 60, 102 entfallen.

In der Beleuchtungseinheit 42 ist zum Beispiel ein optischer Wellenleiter angeordnet, mit dem das Referenzlicht und das Rasterlicht voneinander getrennt werden können. Referenzlicht wird zur Einkopplung zum Beispiel über Lichtleiter 126 zugeführt. Am jeweiligen Lichtleiter 126 ist ein Auskoppelelement 128 angeordnet, aus dem das Referenzlicht vorzugsweise mit sphärischer Wellenfront emittiert. Die Lichtleiter 126 und Auskoppelelemente 128 sind vorzugsweise seitlich nebeneinander in der Ebene des VPH 56 angeordnet.

Das Referenzlicht trifft auf in derselben Ebene angeordnete, in Reihe nebeneinander positionierte Mikrolinsen 130. Dadurch wird eine jeweilige planare Wellenfront bereitgestellt, die durch das VPH 56 propagiert. Ist die jeweilige Bragg-Bedingung für die unterschiedlichen Wellenlängen im VPH 56 erfüllt, wird das Referenzlicht wird in Richtung des Bildsensors 32 gebeugt.

Wie in den vorangegangenen Fällen sind die Wellenfronten 86 der einzelnen Wellenlängen relativ zueinander geneigt und vorzugsweise relativ zur Ebene des Bildsensors 32 geneigt.

Licht, das durch Beugung oder Streuung nicht die Bragg-Bedingung des VPH 56 erfüllt, kann vom Absorberelement 80 absorbiert werden.

Ein Vorteil in der Verwendung von Referenzwellen über das Array der Mikrolinsen 130 oder einem funktionsgleichen multifokalen Element besteht darin, dass eine planare Welle von Referenzlicht mit nahezu konstanter Intensität über die Ebene des VPH 56 ausgekoppelt werden kann. Auf diese Weise kann auch am Rand des Bildsensors 32 eine hinreichende Signalausbeute erzielt werden.

Anstelle des Arrays von Mikrolinsen 130 kann eine Mehrzahl von VPHs zusätzlich eingesetzt werden, deren jedes die Funktion einer der Mikrolinsen 130 ausübt. Diese VPHs könnten in ein optisches Element integriert werden, das beispielsweise körperlich und optisch direkt an die Beleuchtungseinheit 42 und das VPH 56 angekoppelt werden könnte. Auf diese Weise könnten etwaige Zwischenräume, wie zum Beispiel bei den Mikrolinsen 130, vermieden und dadurch optimierte Wellenfelder bereitgestellt werden.

Alternativ kann zum Beispiel ein langgestrecktes Wellenfeld generiert werden, das seitlich auf das VPH 56 auftrifft. Hierbei kommen zum Beispiel Arrays aus GRIN-Linsen zum Einsatz. Auf diese Weise kann eine entlang des VPH 56 erstreckte Phasenfront so kontinuierlich bereitgestellt werden, dass keine Phasensprünge im Übergang zum VPH 56 entstehen.

Durch die seitliche Platzierung der Bildsensoren 30, 32 nebeneinander ergibt sich bei der Vorrichtung 120 eine tiefenabhängige Parallaxe von Rasterlicht und Objektlicht. Aufgrund dessen, dass die Tiefeninformation absolut bestimmt werden kann, besteht dennoch die Möglichkeit, den Bilddatensatz 35 zum Referenzdatensatz zu registrieren.

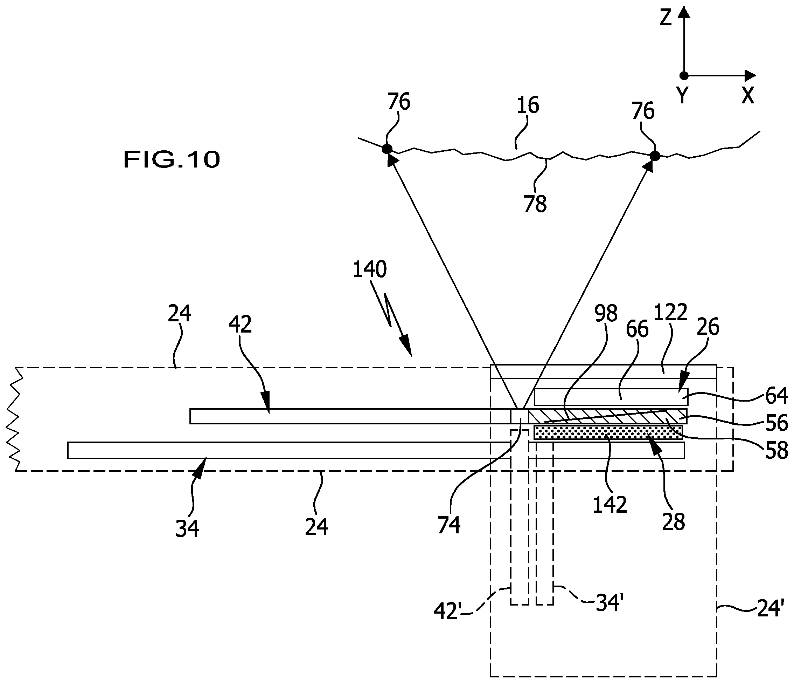

Figur 10 zeigt mit dem Bezugszeichen 140 eine bevorzugte Ausführungsform der erfindungsgemäßen Vorrichtung. Die Vorrichtung 140 ist hochintegriert und derart beschaffen, dass anstelle von zwei Bildsensoren 30, 32 nur ein Bildsensor 142 zum Einsatz kommt. Dieser Bildsensor 142 ist geeignet, um sowohl die NIR-Beiträge des Referenzlichts und Rasterlichts als auch die sichtbaren Beiträge des Objektlichts aufzunehmen.

Auch die Vorrichtung 140 eignet sich für einen Flachaufbau, zum Beispiel in einem tragbaren Kommunikationsgerät oder in intrakorporalen Vorrichtung, beispielsweise in einer Magenpille. Alternativ ist ein schlanker Aufbau denkbar (Bezugszeichen mit Hochkomma).

Eintrittsseitig ist das Eintrittsfenster 122 vorgesehen, gefolgt vom Mikrolinsenarray 66. Sowohl das Objektlicht als auch das Rasterlicht durchqueren das VPH 56. Der Aufbau des VPHs 56 und die Einkopplung des Referenzlichtes wurde bereits vorstehend im Zusammenhang mit der Vorrichtung 120 sowie der diesbezüglichen Abwandlungen beschrieben (Figuren 8 und 9)

Licht gelangt auf den Bildsensor 142, dessen Bildsignale von der Datenverarbeitungseinheit 34 verarbeitet werden.

Um einen möglichst kompakten Aufbau in der Höhenrichtung zu erzielen, ist das optische Element 64 erforderlich. Jedoch kann dieses sich als störend für die Ausbreitung des Rasterlichtes erweisen.

Zur Verbesserung kann als optisches Element 64 eine Amplitudenmaske mit einer Struktur, die wellenlängenabhängig ist, eingesetzt werden. Für das Objektlicht können die Strukturen reflektiv ausgestaltet sein, wohingegen sie für das Rasterlicht transparent sind. In diesem Fall wäre das optische Element 64 für das Rasterlicht im Wesentlichen transparent.

Selbst in dem Fall, dass Amplituden- oder Phasenmasken das Rasterlicht voll beeinflussen, könnte diese "Störung" aus den Bildsignalen für das Rasterlicht herausgerechnet werden. Die diesbezüglichen Dekonvolutions-Verfahren sind dem Fachmann bekannt.

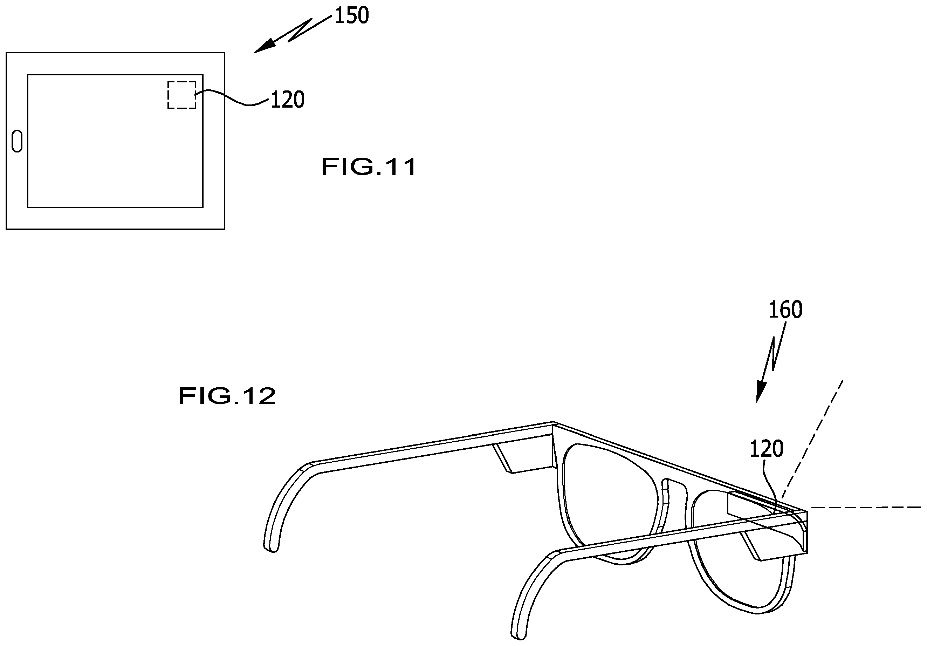

Die Figur 11 zeigt ein mit dem Bezugszeichen 150 belegtes erfindungsgemäßes Kommunikationsgerät, ausgestaltet als Smartphone, mit einer Vorrichtung der vorstehend genannten Art, beispielsweise der Vorrichtung 120.

Die Figur 12 zeigt ein mit dem Bezugszeichen 160 belegtes erfindungsgemäßes Head-mounted-Gerät, ausgestaltet als Datenbrille, mit einer Vorrichtung der vorstehend genannten Art, beispielsweise der Vorrichtung 120 oder 140. Über die Vorrichtung 120 oder 140 könnte die nähere Umgebung hochgenau aufgenommen werden um dann diese Information entweder zu optimieren oder als Hintergrund für ein "Overlay" für erweiterte Realität (AR, Augmented Reality) zu nutzen.

Bei dem Optimierungs-Ansatz wäre es denkbar, ein Buch mit seiner 3D-Topografie so zu lesen, dass die Buchstaben scharf erscheinen und zwar an exakt der gleichen Stelle. Dafür braucht man die Topografie der Seite als Gegenstand 16 und hochaufgelöst die Buchstaben. Diese lassen sich über die Datenbrille dem Sichtfeld überlagern. Die Buchstaben würden ohne störenden Versatz scharf gelesen.

Bei dem "Overlay-Ansatz" würde eine externe Information gemessen mit der Vorrichtung 120 oder 140 auf die tatsächliche Realität angepasst. Im Dentalbereich könnte beispielsweise ein Dentalimplantat an der exakt richtigen Position im Gebiss angezeigt werden, dem Zahnarzt über eine die 3D-AR- Datenbrille visualisiert und gegebenenfalls über eine externe Anzeigeeinheit 38 geteilt.

### Bezugszeichenliste

- 10: Vorrichtung
- 12: Patient
- 14: Benutzer
- 16: Gegenstand
- 18: distaler Abschnitt
- 20: proximaler Abschnitt
- 22: Griffelement
- 24: Gehäuse
- 26: optische Einheit
- 28: Sensoreinheit
- 30, 32: Bildsensor
- 34: Datenverarbeitungseinheit
- 35: Bilddatensatz
- 36: Leitung
- 38: Anzeigeeinheit
- 40: Speichereinheit
- 42: Beleuchtungseinheit
- 44: Lichtquelle
- 46: Lichtquelle
- 48: Eintrittsöffnung
- 50: Austrittsöffnung
- 52: Außenwand
- 54: Filterelement
- 56: Volume-Phase-Hologram (VPH)
- 58: Beugungsgitter
- 60: Strahlteilerwürfel
- 62: Reflexionsschicht
- 64: optisches Element
- 66: Mikrolinsenarray
- 68, 70: Signalleitung
- 72: Lichtleiter
- 74: Auskoppelelement
- 76: Leuchtfleck
- 78: Oberfläche
- 80: Absorberelement
- 82: Lichtleiter
- 84: Auskoppelelement
- 86: Wellenfront
- 88: Achse
- 90: Specklemuster
- 100: Vorrichtung
- 102: Strahlteilerwürfel
- 104: Reflexionsschicht
- 106: Auskoppelelement
- 108: optisches Element
- 110: Hohlspiegel
- 112: Lichtleiter
- 114: Auskoppelelement
- 116: optisches Element
- 118: Sammellinse
- 120: Vorrichtung
- 122: Eintrittsfenster
- 126: Lichtleiter
- 128: Auskoppelelement
- 130: Mikrolinse
- 140: Vorrichtung
- 142: Bildsensor
- 150: Kommunikationsgerät
- 160: Head-mounted-Gerät

## Patentansprüche

1. Optische Vorrichtung zur Untersuchung eines Gegenstandes (16), umfassend
ein Gehäuse (24),
eine im Gehäuse (24) angeordnete optische Einheit (26) für einfallendes Licht,
eine Sensoreinheit (28) mit mindestens einem im Gehäuse (24) angeordneten Bildsensor (30, 32),
eine Datenverarbeitungseinheit (34), die mit der Sensoreinheit (28) gekoppelt ist und Bildsignale des mindestens einen Bildsensors (30, 32) auswertet,
eine zumindest teilweise am oder im Gehäuse (24) angeordnete Beleuchtungseinheit (42) zur Emission von Rasterlicht in Richtung des Gegenstandes (16),
wobei mittels vom Gegenstand (16) reflektierten Rasterlicht und gehäuseinternem Referenzlicht durch digitale optische Holografie ein dreidimensionales Punktraster und diesbezügliche Referenzdatensätze indikativ für eine Lateralinformation und eine Tiefeninformation bereitgestellt werden,
wobei über die Vorrichtung (10; 100; 120; 140) eine Relativbewegung der Vorrichtung (10; 100; 120; 140) und des Gegenstandes (16) erfasst und eine diesbezügliche Bewegungsinformation in Lateral- und/oder Tiefenrichtung erstellt wird,
wobei vom Gegenstand (16) ausgehendes Objektlicht erfasst und zeitlich aufeinanderfolgend ein jeweiliger Bilddatensatz (35) erstellt wird, der relativ zum Referenzdatensatz zur Erstellung eines 3D-Oberflächendatensatzes registriert wird,
wobei einander überlappende Bereiche von zwei oder mehr aufeinanderfolgenden Bilddatensätzen (35) anhand der Bewegungsinformation identifiziert werden und die überlappenden Bereiche durch Integration der diesbezüglichen Bildsignale geglättet werden,
**dadurch gekennzeichnet, dass**
zur Ermittlung der Relativbewegung mindestens ein vom Rasterlicht auf dem Gegenstand (16) erzeugter Leuchtfleck (76) des Punktrasters mit Specklemuster (90) zeitabhängig untersucht wird, wobei eine Verschiebung des Specklemuster (90) innerhalb eines Leuchtflecks (76) festgestellt und hieraus eine Bewegungsinformation in Lateralrichtung abgeleitet wird.

2. Vorrichtung nach Anspruch 1, **dadurch gekennzeichnet, dass** überlappende Bereiche der zwei oder mehr Bilddatensätze (35) durch inverse Bewegung rechnerisch übereinandergelegt werden.

3. Vorrichtung nach einem der voranstehenden Ansprüche, **dadurch gekennzeichnet, dass** eine Mehrzahl von 3D-Oberflächendatensätzen zu einer Gesamtszene des Gegenstandes (16) zusammengesetzt wird, wobei Grenzen zwischen 3D-Oberflächendatensätzen anhand der Bewegungsinformation ermittelt werden.

4. Vorrichtung nach einem der voranstehenden Ansprüche, **dadurch gekennzeichnet, dass** das Rasterlicht und das Referenzlicht ein Spektrum mit einer Mehrzahl diskreter Wellenlängen umfasst und dass eine Bewegungsinformation in Tiefenrichtung und/oder eine absolute Tiefeninformation anhand von Phasendifferenzen der multiplen Wellenlängen ermittelbar ist.

5. Vorrichtung nach einem der voranstehenden Ansprüche, **dadurch gekennzeichnet, dass** zumindest eines der Folgenden gilt:
- das Rasterlicht ist oder umfasst Infrarotlicht und/oder Licht des sichtbaren Spektrums;
- das Objektlicht ist oder umfasst Licht des sichtbaren Spektrums, insbesondere eines zusammenhängenden Spektralbereichs.

6. Vorrichtung nach einem der voranstehenden Ansprüche, **dadurch gekennzeichnet, dass** die Beleuchtungseinheit (42) mindestens einen Lichtleiter (72, 82, 112) für Rasterlicht im Gehäuse (24) umfasst und ein insbesondere distal am Gehäuse (24) angeordnetes optisches Auskoppelelement (74, 84, 114), vorzugsweise dass das Auskoppelelement (74, 84, 114) ein planares Hologramm ist oder umfasst, zur Auffächerung des Rasterlichts in das Punktraster auf dem Gegenstand (16).

7. Vorrichtung nach einem der voranstehenden Ansprüche, **dadurch gekennzeichnet, dass** zumindest eines der Folgenden gilt:
- die Beleuchtungseinheit (42) umfasst eine Lichtquelle (44) zum Bereitstellen des Rasterlichts und des Referenzlichts, die im Gehäuse (24) angeordnet ist;
- die Beleuchtungseinheit (42) umfasst eine Lichtquelle (46) zum Bereitstellen des Objektlichts, die im Gehäuse (24) angeordnet ist.

8. Vorrichtung nach einem der Ansprüche 1 bis 7, **dadurch gekennzeichnet, dass** die Sensoreinheit (28) zwei Bildsensoren (30, 32) umfasst, wobei über ein optisches Element (60, 102, 116) der optischen Einheit (26) Objektlicht auf einen der Bildsensoren (30, 32) und Rasterlicht auf den anderen Bildsensor (30, 32) leitbar ist, vorzugsweise dass die Bildsensoren (30, 32) für das jeweils detektierte Licht sensitiver sind als für das jeweils andere Licht.

9. Vorrichtung nach einem der voranstehenden Ansprüche, **dadurch gekennzeichnet, dass** zwei Bildsensoren (30, 32) vorgesehen sind, die seitlich nebeneinander positioniert sind, insbesondere in einer gemeinsamen Ebene, und von mindestens einem Eintrittsfenster (122) überdeckt sind.

10. Vorrichtung nach einem der voranstehenden Ansprüche, **dadurch gekennzeichnet, dass** zwei Bildsensoren (30, 32) vorgesehen sind, die in im Winkel zueinander ausgerichteten Ebenen angeordnet sind, und dass ein wellenlängensensitives Strahlteilerelement (60, 102) einem jeweiligen Bildsensor (30, 32) in Einfallsrichtung des Objektlichtes bzw. des Rasterlichtes vorgelagert angeordnet ist.

11. Vorrichtung nach einem der voranstehenden Ansprüche, **dadurch gekennzeichnet, dass** die optische Einheit (26) einen für Objektlicht sensitiven Bildsensor (30, 32) in Einfallsrichtung des Objektlichts vorgelagert ein phasenmodulierendes und/oder amplitudenmodulierendes optisches Element (64) umfasst, insbesondere ein Mikrolinsenarray (66), eine Phasenmaske oder eine Amplitudenmaske.

12. Vorrichtung nach einem der voranstehenden Ansprüche, **dadurch gekennzeichnet, dass** die optische Einheit (26) ein VPH (Volume-Phase-Hologram) (56) zur Beugung des Referenzlichts in Richtung des mindestens einen Bildsensors (30, 32, 142) umfasst, vorzugsweise dass das VPH (56) an einer Eintrittsseite der Vorrichtung (10; 100; 120; 140) positioniert und transmittiv für das Objektlicht und/oder das Rasterlicht ist und/oder dass das VPH (56) parallel zu einer Ebene eines Bildsensors (30, 32) ausgerichtet und dem Bildsensor (30, 32) in Einfallsrichtung des Rasterlichts unmittelbar vorgelagert angeordnet ist.

13. Vorrichtung nach einem der voranstehenden Ansprüche, **dadurch gekennzeichnet, dass** die Beleuchtungseinheit (42) zum Erzeugen einer im Wesentlichen planaren Wellenfront (86) des Referenzlichtes oberhalb einer Ebene des mindestens einen Bildsensors (30, 32) eines der Folgenden umfasst:
- eine Mehrzahl von nebeneinander in einer Reihe angeordneten Mikrolinsen (130);
- ein Multi-Volumen-Hologramm;
- ein Array aus GRIN-Linsen.

14. Vorrichtung nach einem der voranstehenden Ansprüche, **dadurch gekennzeichnet, dass** die optische Einheit (26) ein optisches Element (108) zur Aufweitung von Referenzlicht mit als planarer oder im Wesentlichen planarer Wellenfront (86) in Richtung des mindestens einen Bildsensors (30, 32) umfasst, insbesondere dass das optische Element (108) ein Hohlspiegel (110) ist.

15. Vorrichtung nach einem der voranstehenden Ansprüche, **dadurch gekennzeichnet, dass** eines der Folgenden gilt:
- die Vorrichtung (10; 100; 120; 140) ist eine endoskopische Vorrichtung, die zumindest abschnittsweise mit dem Gehäuse (24) in einen Untersuchungsgegenstand einbringbar ist;
- die Vorrichtung (10; 100; 120; 140) ist ein tragbares Kommunikationsgerät (150) oder ist von einem solchen umfasst, insbesondere ein Smartphone oder ein Tablet-Computer;
- die Vorrichtung (10; 100; 120; 140) ist eine Head-mounted-Gerät (160) oder ist von einer solchen umfasst.

## Claims

1. An optical device for examining an object (16), comprising a casing (24),
an optical unit (26) for incident light, arranged in the casing (24),
a sensor unit (28) comprising at least one image sensor (30, 32) arranged in the casing (24),
a data processing unit (34) coupled to the sensor unit (28) evaluating image signals from the at least one image sensor (30, 32),
an illumination unit (42) arranged at least partially on or within the casing (24) for emitting raster light in the direction of the object (16),
wherein, by means of raster light reflected from the object (16) and reference light internal to the casing, a three-dimensional point grid and associated reference data sets indicative of lateral information and depth information are provided via digital optical holography,
wherein, via the device (10; 100; 120; 140), a relative movement of the device (10; 100; 120; 140) and the object (16) is detected and corresponding movement information in the lateral and/or depth direction is generated, wherein object light emanating from the object (16) is detected and a respective image data set (35) is generated successively in time, which is registered relative to the reference data set to generate a 3D surface data set,
wherein overlapping regions of two or more successive image data sets (35) are identified based on the movement information, and the overlapping regions are smoothed by integrating the relevant image signals,
**characterised in that**
to determine the relative movement, at least one light spot (76) of the dot matrix with a speckle pattern (90), generated by the raster light on the object (16), is examined as a function of time, whereby a displacement of the speckle pattern (90) within a light spot (76) is detected and movement information in the lateral direction is derived therefrom.

2. The device according to claim 1, **characterised in that** overlapping regions of the two or more image data sets (35) are computationally superimposed on one another by inverse movement.

3. The device according to one of the preceding claims, **characterised in that** a plurality of 3D surface datasets are combined to form an overall scene of the object (16), wherein boundaries between 3D surface datasets are determined based on the movement information.

4. The device according to one of the preceding claims, **characterised in that** the raster light and the reference light comprise a spectrum with a plurality of discrete wavelengths, and **in that** movement information in the depth direction and/or absolute depth information can be determined based on phase differences between the multiple wavelengths.

5. The device according to any of the preceding claims, **characterised in that** at least one of the following applies:
- the raster light is, or comprises, infrared light and/or light in the visible spectrum;
- the object light is, or comprises, light in the visible spectrum, in particular a contiguous spectral range.

6. The device according to any one of the preceding claims, **characterised in that** the illumination unit (42) comprises at least one light guide (72, 82, 112) for raster light within the casing (24) and an optical decoupling element (74, 84, 114), preferably arranged distally on the casing (24), for spreading the raster light into the dot pattern on the object (16).

7. The device according to any one of the preceding claims, **characterised in that** at least one of the following applies:
- the illumination unit (42) comprises a light source (44) for providing the raster light and the reference light, arranged in the casing (24);
- the illumination unit (42) comprises a light source (46) for providing the object light, which is arranged in the casing (24).

8. The device according to any one of claims 1 to 7, **characterised in that** the sensor unit (28) comprises two image sensors (30, 32), wherein, via an optical element (60, 102, 116) of the optical unit (26), object light can be directed onto one of the image sensors (30, 32) and raster light onto the other image sensor (30, 32), preferably so that the image sensors (30, 32) are more sensitive to the light detected in each case than to the other light.

9. The device according to one of the preceding claims, **characterised in that** two image sensors (30, 32) are provided, which are arranged side by side, in particular in a common plane, and are covered by at least one entrance window (122).

10. The device according to one of the preceding claims, **characterised in that** two image sensors (30, 32) are provided, which are arranged in planes oriented at an angle to one another, and **in that** a wavelength-sensitive beam-splitter element (60, 102) is arranged upstream in the direction of incidence of the object light or the raster light to a respective image sensor (30, 32).

11. The device according to one of the preceding claims, **characterised in that** the optical unit (26) comprises an object light sensitive image sensor (30, 32) and, upstream in the direction of incidence of the object light, a phase-modulating and/or amplitude-modulating optical element (64), in particular a microlens array (66), a phase mask or an amplitude mask.

12. The device according to one of the preceding claims, **characterised in that** the optical unit (26) comprises a VPH (volume-phase hologram) (56) for diffracting the reference light in the direction of the at least one image sensor (30, 32, 142), preferably the VPH (56) is positioned on an input side of the device (10; 100; 120; 140) and is transmissive to the object light and/or the raster light and/or that the VPH (56) is aligned parallel to a plane of an image sensor (30, 32) and is arranged proximately upstream in the direction of incidence of the raster light to the image sensor (30, 32).

13. The device according to one of the preceding claims, **characterised in that** the illumination unit (42) comprises, for generating a substantially planar wavefront (86) of the reference light above a plane of the at least one image sensor (30, 32), one of the following:
- a plurality of microlenses (130) arranged side by side in a row;
- a multi-volume hologram;
- an array of GRIN lenses.

14. The device according to one of the preceding claims, **characterised in that** the optical unit (26) comprises an optical element (108) for expanding reference light with a planar or substantially planar wavefront (86) in the direction of the at least one image sensor (30, 32), in particular the optical element (108) is a concave mirror (110).

15. The device according to any one of the preceding claims, **characterised in that** one of the following applies:
- the device (10; 100; 120; 140) is an endoscopic device which is insertable, at least in sections, into an object under examination together with the casing (24);
- the device (10; 100; 120; 140) is a portable communication device (150) or comprises such a device, in particular a smartphone or a tablet computer;
- the device (10; 100; 120; 140) is a head-mounted device (160) or comprises such a device.

## Revendications

1. Dispositif optique pour l'examen d'un objet (16), comprenant un logement (24),
une unité optique (26) disposée dans le logement (24) pour de la lumière incidente, une unité de capteur (28) avec au moins un capteur d'image (30, 32) disposé dans le logement (24),
une unité de traitement de données (34) qui est couplée à l'unité de capteur (28) et évalue des signaux d'image de l'au moins un capteur d'image (30, 32),
une unité d'éclairage (42) disposée au moins partiellement sur ou dans le logement (24) pour l'émission de lumière de grille en direction de l'objet (16),
dans lequel une grille de points tridimensionnelle et des ensembles de données de référence s'y rapportant indicateurs d'une information latérale et d'une information de profondeur sont mis à disposition au moyen de lumière de grille réfléchie par l'objet (16) et de lumière de référence interne au logement par une holographie optique numérique,
dans lequel un mouvement relatif du dispositif (10 ; 100 ; 120 ; 140) et de l'objet (16) est détecté par le biais du dispositif (10 ; 100 ; 120 ; 140), et une information de mouvement s'y rapportant dans la direction latérale et/ou de profondeur est créée,
dans lequel de la lumière d'objet partant de l'objet (16) est détectée et un ensemble de données d'image respectif (35) est créé successivement dans le temps, lequel est enregistré par rapport à l'ensemble de données de référence pour la création d'un ensemble de données de surface tridimensionnelle,
dans lequel des régions se chevauchant de deux ensembles de données d'image successifs (35) ou plus sont identifiées à l'aide de l'information de mouvement et les régions chevauchantes sont lissées par intégration des signaux d'image s'y rapportant,
**caractérisé en ce que**
au moins un point lumineux (76) de la grille de points généré par la lumière de grille sur l'objet (16) est examiné en fonction du temps avec une figure de speckle (90) pour la détermination du mouvement relatif, dans lequel un décalage de la figure de speckle (90) au sein d'un point lumineux (76) est constaté et une information de mouvement dans la direction latérale en est dérivée.

2. Dispositif selon la revendication 1, **caractérisé en ce que** des régions chevauchantes des deux ensembles de données d'image (35) ou plus sont superposées mathématiquement par mouvement inverse.

3. Dispositif selon une des revendications précédentes, **caractérisé en ce qu'**une pluralité d'ensembles de données de surface tridimensionnelle est réunie en une scène entière de l'objet (16), dans lequel des limites entre des ensembles de données de surface tridimensionnelle sont déterminées à l'aide de l'information de mouvement.

4. Dispositif selon une des revendications précédentes, **caractérisé en ce que** la lumière de grille et la lumière de référence comprennent un spectre avec une pluralité de longueurs d'ondes discrètes, et qu'une information de mouvement dans la direction de profondeur et/ou une information de profondeur absolue peut être déterminée à l'aide de différences de phase des multiples longueurs d'ondes.

5. Dispositif selon une des revendications précédentes, **caractérisé en ce qu'**au moins un des points suivants est valable :
- la lumière de grille est ou comprend de la lumière infrarouge et/ou lumière du spectre visible ;
- la lumière d'objet est ou comprend de la lumière du spectre visible, notamment d'une région spectrale cohérente.

6. Dispositif selon une des revendications précédentes, **caractérisé en ce que** l'unité d'éclairage (42) comprend au moins un guide de lumière (72, 82, 112) pour de la lumière de grille dans le logement (24) et un élément de découplage optique (74, 84, 114) disposé notamment de manière distale sur le logement (24), de préférence que l'élément de découplage (74, 84, 114) est ou comprend un hologramme plan pour la dispersion de la lumière de grille dans la grille de points sur l'objet (16).

7. Dispositif selon une des revendications précédentes, **caractérisé en ce qu'**au moins un des points suivants est valable :
- l'unité d'éclairage (42) comprend une source lumineuse (44) pour la mise à disposition de la lumière de grille et de la lumière de référence qui est disposée dans le logement (24) ;
- l'unité d'éclairage (42) comprend une source lumineuse (46) pour la mise à disposition de la lumière d'objet qui est disposée dans le logement (24).

8. Dispositif selon une des revendications 1 à 7, **caractérisé en ce que** l'unité de capteur (28) comprend deux capteurs d'image (30, 32), dans lequel de la lumière d'objet peut être dirigée vers un des capteurs d'image (30, 32) et de la lumière de grille peut être dirigée vers l'autre capteur d'image (30, 32) par le biais d'un élément optique (60, 102, 116) de l'unité optique (26), de préférence que les capteurs d'image (30, 32) sont plus sensibles pour la lumière détectée respectivement que pour l'autre lumière respective.

9. Dispositif selon une des revendications précédentes, **caractérisé en ce que** deux capteurs d'image (30, 32) sont prévus, lesquels sont positionnés latéralement côte à côte, notamment dans un plan commun, et sont recouverts d'au moins une fenêtre d'entrée (122).

10. Dispositif selon une des revendications précédentes, **caractérisé en ce que** deux capteurs d'image (30, 32) sont prévus, lesquels sont disposés dans des plans orientés à un angle l'un de l'autre, et qu'un élément séparateur de faisceaux (60, 102) sensible à la longueur d'ondes est disposé de manière logée en amont d'un capteur d'image respectif (30, 32) dans la direction d'incidence de la lumière d'objet ou de la lumière de grille.

11. Dispositif selon une des revendications précédentes, **caractérisé en ce que** l'unité optique (26) comprend un élément optique à modulation de phase et/ou modulation d'amplitude (64) de manière logée en amont d'un capteur d'image (30, 32) sensible à la lumière d'objet dans la direction d'incidence de la lumière d'objet, notamment une matrice de microlentilles (66), un masque de phase ou un masque d'amplitude.

12. Dispositif selon une des revendications précédentes, **caractérisé en ce que** l'unité optique (26) comprend un VPH (hologramme de phase volumique) (56) pour la diffraction de la lumière de référence en direction de l'au moins un capteur d'image (30, 32, 142), de préférence que le VPH (56) est positionné sur un côté d'entrée du dispositif (10 ; 100 ; 120 ; 140) et est transmissif pour la lumière d'objet et/ou la lumière de grille et/ou que le VPH (56) est orienté parallèlement à un plan d'un capteur d'image (30, 32) et est disposé de manière directement logée en amont du capteur d'image (30, 32) dans la direction d'incidence de la lumière de grille.

13. Dispositif selon une des revendications précédentes, **caractérisé en ce que** l'unité d'éclairage (42) comprend un des éléments suivants pour la génération d'un front d'onde essentiellement plan (86) de la lumière de référence au-dessus d'un plan de l'au moins un capteur d'image (30, 32) :
- une pluralité de microlentilles (130) disposées côte à côte en une rangée ;
- un hologramme multivolumique ;
- une matrice de lentilles GRIN.

14. Dispositif selon une des revendications précédentes, **caractérisé en ce que** l'unité optique (26) comprend un élément optique (108) pour l'élargissement de la lumière de référence avec un front d'onde plan ou essentiellement plan (86) en direction de l'au moins un capteur d'image (30, 32), notamment que l'élément optique (108) est un miroir creux (110).

15. Dispositif selon une des revendications précédentes, **caractérisé en ce qu'**un des points suivants est valable :
- le dispositif (10 ; 100 ; 120 ; 140) est un dispositif endoscopique qui peut être introduit au moins par sections avec le logement (24) dans un objet d'examen ;
- le dispositif (10 ; 100 ; 120 ; 140) est un appareil de communication portable (150) ou est compris par un tel appareil, notamment un smartphone ou une tablette ;
- le dispositif (10 ; 100 ; 120 ; 140) est un casque à réalité virtuelle (160) ou est compris par un tel casque.
